# EUROPEAN PATENT SPECIFICATION

(11) **EP 1 379 693 B1**
(45) Date of publication and mention of the grant of the patent: **20.05.2009**
(21) Application number: 02725590.0
(22) Date of filing: 27.03.2002
(51) Int. Cl.: C12Q 1/68

(54) **BIO-BARCODES BASED ON OLIGONUCLEOTIDE-MODIFIED PARTICLES**
AUF OLIGONUKLEOTID-MODIFIZIERTEN PARTIKELN BASIERENDE BIO-STRICHCODES
CODES-BARRES BIOLOGIQUES BASES SUR DES PARTICULES A MODIFICATION OLIGONUCLEOTIDIQUE

(30) Priority: 28.03.2001 US 820279; 28.03.2001 WO PCT/US01/10071; 13.11.2001 US 350560 P
(43) Date of publication of application: 14.01.2004
(73) Proprietor: Nanosphere, Inc., Northbrook, Illinois 60062 (US)
(72) Inventor: MIRKIN, Chad, A., Willmette, IL 60091 (US); PARK, So-Jung, Auston, TX 78731-3006 (US); NAM, Jwa-Miin, Evanston, IL 60201 (US)
(74) Representative: Grund, Martin
(86) International application number: PCT/US2002/011158
(87) International publication number: WO 2002/079490

(56) References cited:
- WO-A-89/06801
- WO-A-90/02205
- WO-A-98/04740
- US-B1- 6 214 560
- US-B1- 6 264 825

## Description

### FIELD OF THE INVENTION:

The present invention relates to a screening method for detecting for the presence or absence of one or more proteins, e.g., antibodies, in a sample. In particular, the present invention relates to a method that utilizes reporter oligonucleotides as biochemical barcodes for detecting multiple protein structures in one solution.

### BACKGROUND OF THE INVENTION:

The detection of proteins is important for both molecular biology research and medical applications. Diagnostic methods based on fluorescence, mass spectroscopy, gell electrophoresis, laser scanning and electrochemistry are now available for identifying a variety of protein structures. ¹⁻⁴ Antibody-based reactions are widely used to identify the genetic protein variants of blood cells, diagnose diseases, localize molecular probes in tissue, and purify molecules or effect separation processes.⁵ For medical diagnostic applications (e.g. malaria and HIV), antibody tests such as the enzyme-linked immunosorbent assay, Western blotting, and indirect fluorescent antibody tests are extremely useful for identifying single target protein structures. ^{6,7} Rapid and simultaneous sample screening for the presence of multiple antibodies would be beneficial in both research and clinical applications. However, it is difficult, expensive, and time-consuming to simultaneously detect several protein structures in one solution under homogeneous assay conditions using the aforementioned related protocols.

WO8906801 discloses a method for the qualitative or quantitative determination of an analyte in a test sample wherein a labelled reagent is caused to be immobilised in bound form on a solid phase to provide an indication of the presence or quantity of the analyte in the sample.

### SUMMARY OF THE INVENTION:

The present invention relates to methods, compositions, and kits that utilizes oligonucleotides as biochemical barcodes for detecting multiple protein structures in one solution. The approach takes advantage of protein recognition elements functionalized with oligonucleotide strands and the previous observation that hybridization events that result in the aggregation of gold nanoparticles can significantly alter their physical properties (e.g. optical, electrical, mechanical).⁸⁻¹² The general idea is that each protein recognition element can be encoded with a different oligonucleotide sequence with discrete and tailorable hybridization and melting properties and a physical signature associated with the nanoparticles that changes upon melting to decode a series of analytes in a multi-analyte assay.

In one embodiment of the invention, a method is provided for detecting for the presence of a target analyte in a sample comprising:
providing a particle complex probe comprising a particle having oligonucleotides bound thereto, a DNA barcode, and a oligonucleotide having bound thereto a specific binding complement to a target analyte, wherein the DNA barcode has a sequence having at least two portions, at least some of the oligonucleotides attached to the particle have a sequence that is complementary to a first portion of a DNA barcode, the oligonucleotides having bound thereto a specific binding complement have a sequence that is complementary to a second portion of a DNA barcode, and wherein the DNA barcode is hybridized at least to some of the oligonucleotides attached to the particle and to the oligonucleotides having bound thereto the specific binding complement;
contacting the sample with a particle complex probe under conditions effective to allow specific binding interactions between the analyte and the particle complex probe and to form an aggregated complex in the presence of analyte; and
observing whether aggregate formation occurred.

In the presence of target analyte, aggregates are produced as a result of the binding interactions between the particle complex probe and the target analyte. The aggregates may be detected by any suitable means.

In another embodiment of the invention, a method is provided for detecting for the presence of one or more target analytes in a sample comprising:
providing one or more types of particle complex probe comprising a particle having oligonucleotides bound thereto, a DNA barcode, and a oligonucleotide having bound thereto a specific binding complement to a specific target analyte, wherein (i) the DNA barcode has a sequence having at least two portions, (ii) at least some of the oligonucleotides attached to the particle have a sequence that is complementary to a first portion of a DNA barcode,(iii) the oligonucleotides having bound thereto a specific binding complement have a sequence that is complementary to a second portion of a DNA barcode, and (iv) the DNA barcode in each type of particle complex probe has a sequence that is different and that serves as an identifier for a particular target analyte;
contacting the sample with a particle complex probe under conditions effective to allow specific binding interactions between the analyte and the particle complex probe and to form an aggregated complex in the presence of analyte;
isolating aggregated complexes; and
analyzing the aggregated complexes to determine the presence of one or more DNA barcodes having different sequences.

Each type of particle complex probe contains a predetermined reporter oligonucleotde or barcode for a particular target analyte. In the presence of target analyte, nanoparticle aggregates are produced as a result of the binding interactions between the nanoparticle complex and the target analyte. These aggregates can be isolated and analyzed by any suitable means, e.g., thermal denaturation, to detect the presence of one or more different types of reporter oligonucleotides.

In yet another embodiment of the invention, a method is provided for detecting for the presence of a target analyte in a sample comprising:
providing a particle complex probe comprising a particle having oligonucleotides bound thereto, a DNA barcode, and a oligonucleotide having bound thereto a specific binding complement to a target analyte, wherein the DNA barcode has a sequence having at least two portions, at least some of the oligonucleotides attached to the particle have a sequence that is complementary to a first portion of a DNA barcode, the oligonucleotides having bound thereto a specific binding complement have a sequence that is complementary to a second portion of a DNA barcode, and wherein the DNA barcode is hybridized at least to some of the oligonucleotides attached to the particle and to the oligonucleotides having bound thereto the specific binding complement;
contacting the sample with a particle complex probe under conditions effective to allow specific binding interactions between the analyte and the particle complex probe and to form an aggregated complex in the presence of analyte;
isolating the aggregated complex and subjecting the aggregated complex to conditions effective to dehybridize the aggregated complex and to release the DNA barcode;
isolating the DNA barcode; and
detecting for the presence of DNA barcode.

In the presence of target analyte, nanoparticle aggregates are produced as a result of the binding interactions between the nanoparticle complex and the target analyte. These aggregates are isolated and subject to conditions effective to dehybridize the aggregate and to release the reporter oligonucleotide. The reporter oligonucleotide is then isolated. If desired, the reporter oligonucleotide may be amplified by any suitable means including PCR amplification. Analyte detection occurs indirectly by ascertaining for the presence of reporter oligonucleotide or biobarcode by any suitable means such as a DNA chip.

In yet another embodiment of the invention, a method for detecting for the presence of one or more target analytes in a sample comprising:
providing one or more types of particle complex probe comprising a particle having oligonucleotides bound thereto, a DNA barcode, and a oligonucleotide having bound thereto a specific binding complement to a specific target analyte, wherein (i) the DNA barcode has a sequence having at least two portions, (ii) at least some of the oligonucleotides attached to the particle have a sequence that is complementary to a first portion of a DNA barcode,(iii) the oligonucleotides having bound thereto a specific binding complement have a sequence that is complementary to a second portion of a DNA barcode, and (iv) the DNA barcode in each type of particle complex probe has a sequence that is different and that serves as an identifier for a particular target analyte;
contacting the sample with a particle complex probe under conditions effective to allow specific binding interactions between the analyte and the particle complex probe and to form aggregated complexes in the presence of one or more analytes;
isolating the aggregated complexes and subjecting the aggregated complexes to conditions effective to dehybridize the aggregated complexes and to release the DNA barcodes;
isolating the DNA barcodes; and
detecting for the presence of one or more DNA barcodes having different sequences, wherein the identification of a particular DNA barcode is indicative of the presence of a specific target analyte in the sample.

In the presence of one or more target analyte, aggregates are produced as a result of the binding interactions between the particle complex probe and the target analyte. These aggregates are isolated and subject to conditions effective to dehybridize the aggregate and to release the reporter oligonucleotides. The reporter oligonucleotides is then isolated. If desired, the reporter oligonucleotide may be amplified by any suitable means including PCR amplification. Analyte detection occurs indirectly by ascertaining for the presence of reporter oligonucleotide or biobarcode by any suitable means such as a DNA chip.

In yet another embodiment of the invention, a method for detecting for the presence of one or more antibodies in a sample comprising:
providing one or more types of particle complex probe comprising a particle having oligonucleotides bound thereto, a DNA barcode, and a oligonucleotide having bound thereto a hapten to a specific target antibody, wherein (i) the DNA barcode has a sequence having at least two portions, (ii) at least some of the oligonucleotides attached to the particle have a sequence that is complementary to a first portion of a DNA barcode,(iii) the oligonucleotides having bound thereto a hapten to a specific target antibody have a sequence that is complementary to a second portion of a DNA barcode, and (iv) the DNA barcode in each type of particle complex probe has a sequence that is different and that serves as an identifier for a particular target antibody;
contacting the sample with a particle complex probe under conditions effective to allow specific binding interactions between the antibody and the particle complex probe and to form aggregated complexes in the presence of one or more target antibodies;
isolating the aggregated complexes and subjecting the aggregated complexes to conditions effective to dehybridize the aggregated complexes and to release the DNA barcodes;
isolating the DNA barcodes; and
detecting for the presence of one or more DNA barcodes having different sequences, wherein the identification of a particular DNA barcode is indicative of the presence of a specific target antibody.

The invention also provides a method is provided for detecting for the presence of a target analyte in a sample which entails generating a particle complex probe in situ. In one embodiment of the invention, a method for detecting for the presence of target analytes comprises:
providing particles having oligonucleotides bound thereto, DNA barcodes, and oligonucleotides having bound thereto a specific binding complement to a target analyte, wherein the DNA barcodes have a sequence with at least two portions, at least some of the oligonucleotides attached to the particles have a sequence that is complementary to a first portion of a DNA barcode, the oligonucleotides having bound thereto a specific binding complement have a sequence that is complementary to a second portion of a DNA barcodes
contacting the sample with a particle complex probe under conditions effective to allow hybridization of the DNA barcode to at least to some of the oligonucleotides attached to the particle and to the oligonucleotides having bound thereto the specific binding complement and to allow specific binding interactions between the analyte and the oligonucleotides having bound thereto a specific binding complement to the analyte, said contacting resulting in the formation of an aggregated complex in the presence of analyte; and
observing whether aggregate formation occurred.

In another embodiment of the invention, a method is provided for detecting for the presence of one or more target analytes in a sample comprising:
providing one or more types of particles having oligonucleotides bound thereto, one or more types of DNA barcodes, and one or more types of oligonucleotides having bound thereto a specific binding complement to a specific target analyte, wherein (i) each type of DNA barcode has a sequence having at least two portions, (ii) at least some of the oligonucleotides attached to the particle have a sequence that is complementary to a first portion of one or more types of DNA barcode,(iii) each type of oligonucleotide having bound thereto a specific binding complement have a sequence that is complementary to a second portion of a type of DNA barcode, and (iv) each type of DNA barcode serves as an identifier for a particular target analyte and has a sequence that is different from another type of DNA barcode;
contacting the sample with one or more types of particles having oligonucleotides bound thereto, one or more types of DNA barcodes, and one or more types of oligonucleotides having bound thereto a specific binding complement to a specific target analyte, under conditions effective to allow hybridization of each type of DNA barcodes at least to some of the oligonucleotides attached to the particles and to a type of oligonucleotides having bound thereto the specific binding complement and to allow specific binding interactions between a specific target analyte and a type of oligonucleotides having bound thereto a specific binding complement to the specific target analyte, said contacting resulting in the formation of aggregated complexes in the presence of one or more specific target analytes;
isolating aggregated complexes; and
analyzing the aggregated complexes to determine the presence of one or more DNA barcode, where the presence of a particular DNA barcode is indicative of the presence of a specific target analyte in the sample.

In yet another embodiment of the invention, a method is provided for detecting for the presence of a target analyte in a sample comprising:
providing particles having oligonucleotides bound thereto, DNA barcodes, and oligonucleotides having bound thereto a specific binding complement to a target analyte, wherein the DNA barcodes have a sequence with at least two portions, at least some of the oligonucleotides attached to the particles have a sequence that is complementary to a first portion of a DNA barcode, the oligonucleotides having bound thereto a specific binding complement have a sequence that is complementary to a second portion of a DNA barcodes;
contacting the sample with a particle complex probe under conditions effective to allow hybridization of the DNA barcode to at least to some of the oligonucleotides attached to the particle and to the oligonucleotides having bound thereto the specific binding complement and to allow specific binding interactions between the analyte and the oligonucleotides having bound thereto a specific binding complement to the analyte, said contacting resulting in the formation of an aggregated complex in the presence of analyte;
isolating the aggregated complex and subjecting the aggregated complex to conditions effective to dehybridize the aggregated complex and to release the DNA barcode; ,
isolating the DNA barcode; and
detecting for the presence of DNA barcode.

In yet another embodiment of the invention, a method is provided for detecting for the presence of one or more target analytes in a sample comprising:
providing one or more types of particles having oligonucleotides bound thereto, one or more types of DNA barcodes, and one or more types of oligonucleotides having bound thereto a specific binding complement to a specific target analyte, wherein (i) each type of DNA barcode has a sequence having at least two portions, (ii) at least some of the oligonucleotides attached to the particle have a sequence that is complementary to a first portion of one or more types of DNA barcode,(iii) each type of oligonucleotide having bound thereto a specific binding complement have a sequence that is complementary to a second portion of a type of DNA barcode, and (iv) each type of DNA barcode serves as an identifier for a particular target analyte and has a sequence that is different from another type of DNA barcode;
contacting the sample with one or more types of particles having oligonucleotides bound thereto, one or more types of DNA barcodes, and one or more types of oligonucleotides having bound thereto a specific binding complement to a specific target analyte, under conditions effective to allow hybridization of each type of DNA barcodes at least to some of the oligonucleotides attached to the particles and to a type of oligonucleotides having bound thereto the specific binding complement and to allow specific binding interactions between a specific target analyte and a type of oligonucleotides having bound thereto a specific binding complement to the specific target analyte, said contacting resulting in the formation of aggregated complexes in the presence of one or more specific target analytes;
isolating the aggregated complexes and subjecting the aggregated complexes to conditions effective to dehybridize the aggregated complexes and to release the DNA barcodes;
isolating the DNA barcodes; and
detecting for the presence of one or more DNA barcodes having different sequences, wherein the identification of a particular DNA barcode is indicative of the presence of a specific target analyte.

In yet another embodiment of the invention, a method is provided for detecting for the presence of one or more antibodies in a sample comprising:
providing one or more types of particles having oligonucleotides bound thereto, one or more types of DNA barcodes, and one or more types of oligonucleotides having bound thereto a hapten to a specific antibody, wherein (i) each type of DNA barcode has a sequence having at least two portions, (ii) at least some of the oligonucleotides attached to the particle have a sequence that is complementary to a first portion of one or more types of DNA barcode,(iii) each type of oligonucleotide having bound thereto a hapten to a specific antibody has a sequence that is complementary to a second portion of a type of DNA barcode, and (iv) each type of DNA barcode serves as an identifier for a particular target antibody and has a sequence that is different from another type of DNA barcode;
contacting the sample with one or more types of particles having oligonucleotides bound thereto, one or more types of DNA barcodes, and one or more types of oligonucleotides having bound thereto a hapten to a specific target antibody, under conditions effective to allow hybridization of each type of DNA barcodes at least to some of the oligonucleotides attached to the particles and to a type of oligonucleotides having bound thereto the hapten and to allow specific binding interactions between a specific target antibody and a type of oligonucleotides having bound thereto a hapten to the specific target antibody, said contacting resulting in the formation of aggregated complexes in the presence of one or more specific target antibodies;
isolating the aggregated complexes and subjecting the aggregated complexes to conditions effective to dehybridize the aggregated complexes and to release the DNA barcodes;
isolating the DNA barcodes; and
detecting for the presence of one or more DNA barcodes having different sequences, wherein the identification of a particular DNA barcode is indicative of the presence of a specific target antibody.

The invention also provides kits for target analyte detection. In one embodiment of the invention, a kit is provided for detecting a target analyte in a sample, the kit comprising at least one container including particle complex probes comprising a particle having oligonucleotides bound thereto, a DNA barcode, and a oligonucleotide having bound thereto a specific binding complement to a target analyte, wherein the DNA barcode has a sequence having at least two portions, at least some of the oligonucleotides attached to the particle have a sequence that is complementary to a first portion of a DNA barcode, the oligonucleotides having bound thereto a specific binding complement have a sequence that is complementary to a second portion of a DNA barcode, and wherein the DNA barcode is hybridized to at least to some of the oligonucleotides attached to the particle and to the oligonucleotides having bound thereto the specific binding complement, and an optional substrate for observing a detectable change.

In another embodiment of the invention, a kit is provided for detecting one or more target analytes in a sample, the kit comprising at least one or more containers, container holds a type of particle complex probe comprising a particle having oligonucleotides bound thereto, a DNA barcode, and a oligonucleotide having bound thereto a specific binding complement to a specific target analyte, wherein (i) the DNA barcode has a sequence having at least two portions, (ii) at least some of the oligonucleotides attached to the particle have a sequence that is complementary to a first portion of a DNA barcode,(iii) the oligonucleotides having bound thereto a specific binding complement have a sequence that is complementary to a second portion of a DNA barcode, and (iv) the DNA barcode in each type of particle complex probe has a sequence that is different and that serves as an identifier for a particular target analyte; wherein the kit optionally includes a substrate for observing a detectable change.

In yet another embodiment of the invention, a kit is provided for the detection of a target analyte, the kit includes at least one pair of containers and an optional substrate for observing a detectable change,
the first container of the pair includes particle probe comprising a particle having oligonucleotides bound thereto and a DNA barcode having a sequence of at least two portions, wherein at least some of the oligonucleotides attached to the particle have a sequence that is complementary to a first portion of a DNA barcode;
the second container of the pair includes an oligonucleotide having a sequence that is complementary to a second portion of the DNA barcode, the oligonucleotide having a moiety that can be used to covalently link a specific binding pair complement of a target analyte.

In yet another embodiment of the invention, a kit is provided for the detection of multiple target analytes in a sample, the kit includes at least two or more pairs of containers,
the first container of each pair includes particle complex probes having particles having oligonucleotides bound thereto and a DNA barcode having a sequence of at least two portions, wherein at least some of the oligonucleotides bound to the particles have a sequence that is complementary to a first portion of a DNA barcode having at least two portions; and
the second container of each pair contains a oligonucleotide having a sequence that is complementary to a second portion of the DNA barcode, the oligonucleotide having a moiety that can be used to covalently link a specific binding pair complement of a target analyte,
wherein the DNA barcode for type of particle complex probe has a sequence that is different and that serves as an identifer for a target analyte and wherein the kit optionally include a substrate for observing a detectable change.

In yet another embodiment of the invention, a kit is provided for the detection of multiple target analytes in a sample, the kit includes a first container and at least two or more pairs of containers,
the first container includes particle complex probes having particles having oligonucleotides bound thereto;
the first container of the pair includes a DNA barcode having a sequence of at least two portions, wherein at least some of the oligonucleotides bound to the particles have a sequence that is complementary to a first portion of the DNA barcode; and
the second container of each pair contains a oligonucleotide having a sequence that is complementary to a second portion of the DNA barcode, the oligonucleotide having a moiety that can be used to covalently link a specific binding pair complement of a target analyte,
wherein the DNA barcode present in the first container of each pair of containers serves as an identifer for a target analyte and has a sequence that is different from a DNA barcode in another pair of containers, and wherein the kite optionally include a substrate for observing a detectable change.

In yet another embodiment of the invention, the particle of any of the foregoing kits may comprise a nanoparticle such as metal, semiconductor, insulator, or magnetic nanoparticles, preferably gold nanoparticles.

The invention also includes a system for detecting one or more target analytes in a sample comprising:
one or more types of particle complex probes, each particle complex probe comprising a particle having oligonucleotides bound thereto, a DNA barcode, and a oligonucleotide having bound thereto a specific binding complement to a specific target analyte, wherein (i) the DNA barcode has a sequence having at least two portions, (ii) at least some of the oligonucleotides attached to the particle have a sequence that is complementary to a first portion of a DNA barcode,(iii) the oligonucleotide having bound thereto a specific binding complement have a sequence that is complementary to a second portion of a DNA barcode, and (iv) the DNA barcode in each type of particle complex probe has a sequence that is different and that serves as an identifier for a particular target analyte.

The particle in the system preferably comprises a nanoparticle such as metal, semiconductor, insulator, or magnetic nanoparticles, preferably gold nanoparticles.

The invention also comprises a particle complex probe comprising a particle having oligonucleotides bound thereto, a DNA barcode, and a oligonucleotide having bound thereto a specific binding complement to a target analyte, wherein the DNA barcode has a sequence having at least two portions, at least some of the oligonucleotides attached to the particle have a sequence that is complementary to a first portion of a DNA barcode, the oligonucleotides having bound thereto a specific binding complement have a sequence that is complementary to a second portion of a DNA barcode, and wherein the DNA barcode is hybridized at least to some of the oligonucleotides attached to the particle and to the oligonucleotides having bound thereto the specific binding complement. The particle in the probe preferably comprises a nanoparticle such as metal, semiconductor, insulator, or magnetic nanoparticles, preferably gold nanoparticles.

The invention also includes an oligonucleotide sequence having bound thereto a specific target complement to a target analyte.

The invention also includes a DNA barcode comprising a oligonucleotide sequence that serves as an identifier for the presence of a specific target analyte.

The invention also includes two or more DNA barcodes comprising an oligonucleotide sequence, each DNA barcode having a different oligonucleotide sequence and serving as an identifier for the presence of a specific target analyte.

As used herein, a "type of" nanoparticles, conjugates, particles, latex microspheres, etc. having oligonucleotides attached thereto refers to a plurality of that item having the same type(s) of oligonucleotides attached to them. "Nanoparticles having oligonucleotides attached thereto" or "Nanoparticles having oligonucleotides attached thereto" are also sometimes referred to as "nanoparticle-oligonucleotide conjugates" or, in the case of the detection methods of the invention, "nanoparticle-oligonucleotide probes," "nanoparticle probes," or just "probes."

The term "nanoparticle complex" or "nanoparticle complex probe" refers to a conjugate comprised of nanoparticle-oligonucleotide conjugates, a reporter oligonucleotide, and an oligonucleotide having bound thereto a specific binding complement to a target analyte.

The term "analyte" refers to the compound or composition to be detected, including drugs, metabolites, pesticides, pollutants, and the like.. The analyte can be comprised of a member of a specific binding pair (sbp) and may be a ligand, which is monovalent (monoepitopic) or polyvalent (polyepitopic), usually antigenic or haptenic, and is a single compound or plurality of compounds which share at least one common epitopic or determinant site. The analyte can be a part of a cell such as bacteria or a cell bearing a blood group antigen such as A, B, D, etc., or an HLA antigen or a microorganism, e.g., bacterium, fungus, protozoan, or virus.

The polyvalent ligand analytes will normally be poly(amino acids), i.e., polypeptides and proteins, polysaccharides, nucleic acids, and combinations thereof. Such combinations include components of bacteria, viruses, chromosomes, genes, mitochondria, nuclei, cell membranes and the like.

For the most part, the polyepitopic ligand analytes to which the subject invention can be applied will have a molecular weight of at least about 5,000, more usually at least about 10,000. In the poly(amino acid) category, the poly(amino acids) of interest will generally be from about 5,000 to 5,000,000 molecular weight, more usually from about 20,000 to 1,000,000 molecular weight; among the hormones of interest, the molecular weights will usually range from about 5,000 to 60,000 molecular weight.

A wide variety of proteins may be considered as to the family of proteins having similar structural features, proteins having particular biological functions, proteins related to specific microorganisms, particularly disease causing microorganisms, etc. Such proteins include, for example, immunoglobulins, cytokines, enzymes, hormones, cancer antigens, nutritional markers, tissue specific antigens, etc.

The types of proteins, blood clotting factors, protein hormones, antigenic polysaccharides, microorganisms and other pathogens of interest in the present invention are specifically disclosed in U.S. Pat. No. 4,650,770.

The monoepitopic ligand analytes will generally be from about 100 to 2,000 molecular weight, more usually from 125 to 1,000 molecular weight.

The analyte may be a molecule found directly in a sample such as a body fluid from a host. The sample can be examined directly or may be pretreated to render the analyte more readily detectible. Furthermore, the analyte of interest may be determined by detecting an agent probative of the analyte of interest such as a specific binding pair member complementary to the analyte of interest, whose presence will be detected only when the analyte of interest is present in a sample. Thus, the agent probative of the analyte becomes the analyte that is detected in an assay. The body fluid can be, for example, urine, blood, plasma, serum, saliva, semen, stool, sputum, cerebral spinal fluid, tears, mucus, and the like.

The term "specific binding pair (sbp) member" refers to one of two different molecules, having an area on the surface or in a cavity which specifically binds to and is thereby defined as complementary with a particular spatial and polar organization of the other molecule. The members of the specific binding pair are referred to as ligand and receptor (antiligand). These will usually be members of an immunological pair such as antigen-antibody, although other specific binding pairs such as biotin-avidin, hormones-hormone receptors, nucleic acid duplexes, IgG-protein A, polynucleotide pairs such as DNA-DNA, DNA-RNA, and the like are not immunological pairs but are included in the invention and the definition of sbp member.

The term "ligand" refers to any organic compound for which a receptor naturally exists or can be prepared. The term ligand also includes ligand analogs, which are modified ligands, usually an organic radical or analyte analog, usually of a molecular weight greater than 100, which can compete with the analogous ligand for a receptor, the modification providing means to join the ligand analog to another molecule. The ligand analog will usually differ from the ligand by more than replacement of a hydrogen with a bond which links the ligand analog to a hub or label, but need not. The ligand analog can bind to the receptor in a manner similar to the ligand. The analog could be, for example, an antibody directed against the idiotype of an antibody to the ligand.

The term "receptor" or "antiligand" refers to any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, nucleic acids, avidin, protein A, barstar, complement component C1q, and the like. Avidin is intended to include egg white avidin and biotin binding proteins from other sources, such as streptavidin.

The term "specific binding" refers to the specific recognition of one of two different molecules for the other compared to substantially less recognition of other molecules. Generally, the molecules have areas on their surfaces or in cavities giving rise to specific recognition between the two molecules. Exemplary of specific binding are antibody-antigen interactions, enzyme-substrate interactions, polynucleotide interactions, and so forth.

The term "non-specific binding" refers to the non-covalent binding between molecules that is relatively independent of specific surface structures. Non-specific binding may result from several factors including hydrophobic interactions between molecules.

The term "antibody" refers to an immunoglobulin which specifically binds to and is thereby defined as complementary with a particular spatial and polar organization of another molecule. The antibody can be monoclonal or polyclonal and can be prepared by techniques that are well known in the art such as immunization of a host and collection of sera (polyclonal) or by preparing continuous hybrid cell lines and collecting the secreted protein (monoclonal), or by cloning and expressing nucleotide sequences or mutagenized versions thereof coding at least for the amino acid sequences required for specific binding of natural antibodies. Antibodies may include a complete immunoglobulin or fragment thereof, which immunoglobulins include the various classes and isotypes, such as IgA, IgD, IgE, IgG1, IgG2a, IgG2b and IgG3, IgM, etc. Fragments thereof may include Fab, Fv and F(ab').sub.2, Fab', and the like. In addition, aggregates, polymers, and conjugates of immunoglobulins or their fragments can be used where appropriate so long as binding affinity for a particular molecule is maintained.

### BRIEF DESCRIPTION OF THE FIGURES

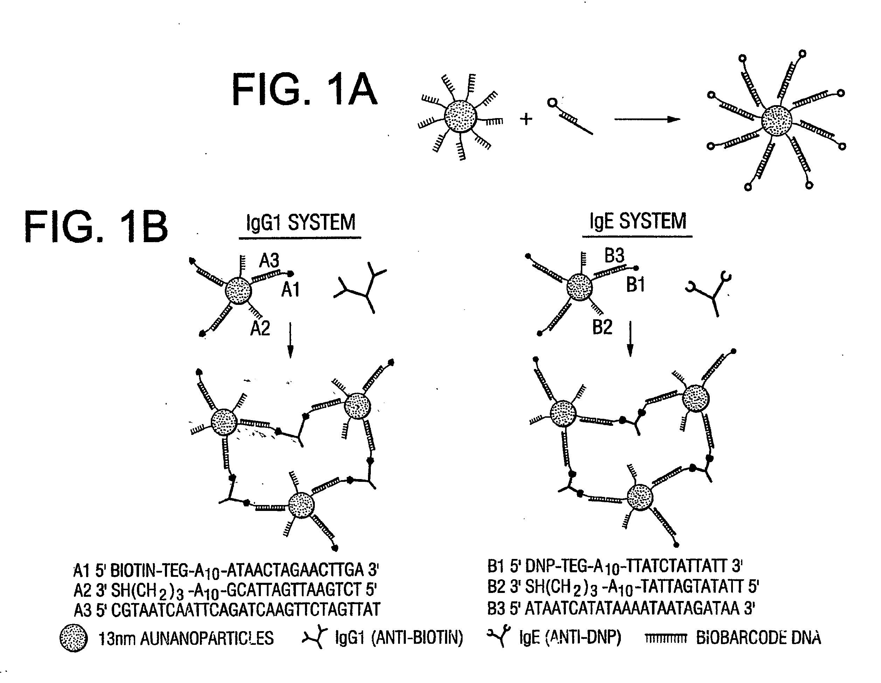

Figure 1 illustrates a DNA/Au nanoparticle-based protein detection scheme. (A) Preparation of hapten-modified nanoparticle probes. (B) Protein detection using protein binding probes. Notice that there are nine G,C pairs in sequence A and there are only two G,C pairs in sequence B.

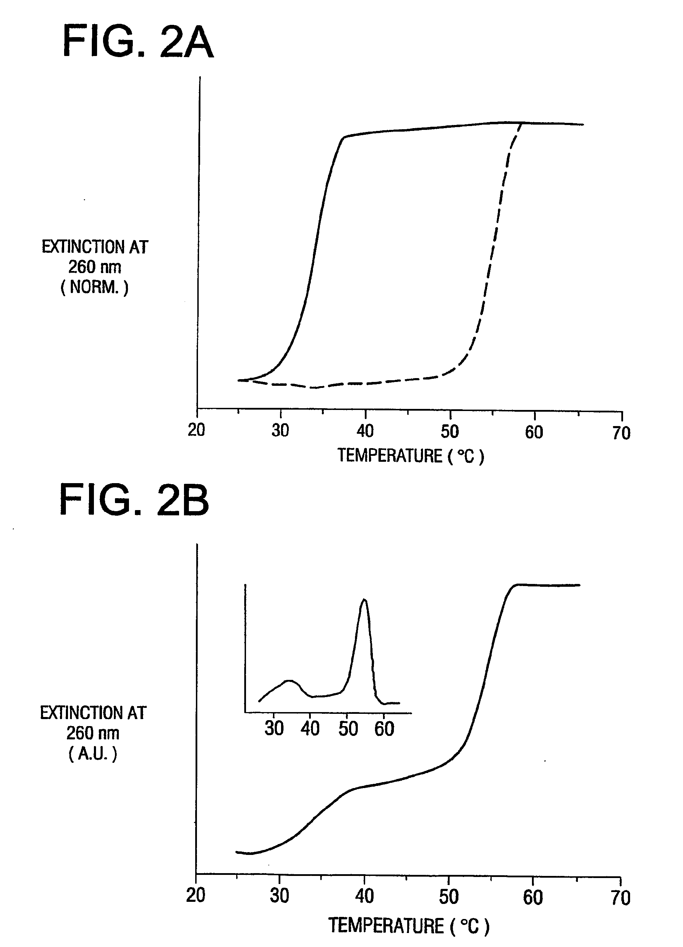

Figure 2 illustrates thermal denaturation profiles for Au nanoparticle aggregates linked by DNA and proteins. Extinction at 260 nm was monitored as a function of increasing temperature (1 °C/min, 1 min holding time). Each UV-Vis spectrum was measured under constant stirring to suspend the aggregates. All the aggregates were suspended in 1 ml of 0.3 M PBS prior to performing the melting analyses. A) Two probes with one target antibody present IgE (-), IgG1 (---)); all data have been normalized; (B) Two probes with both target antibodies present. Inset; first derivative of the thermal denaturation curve.

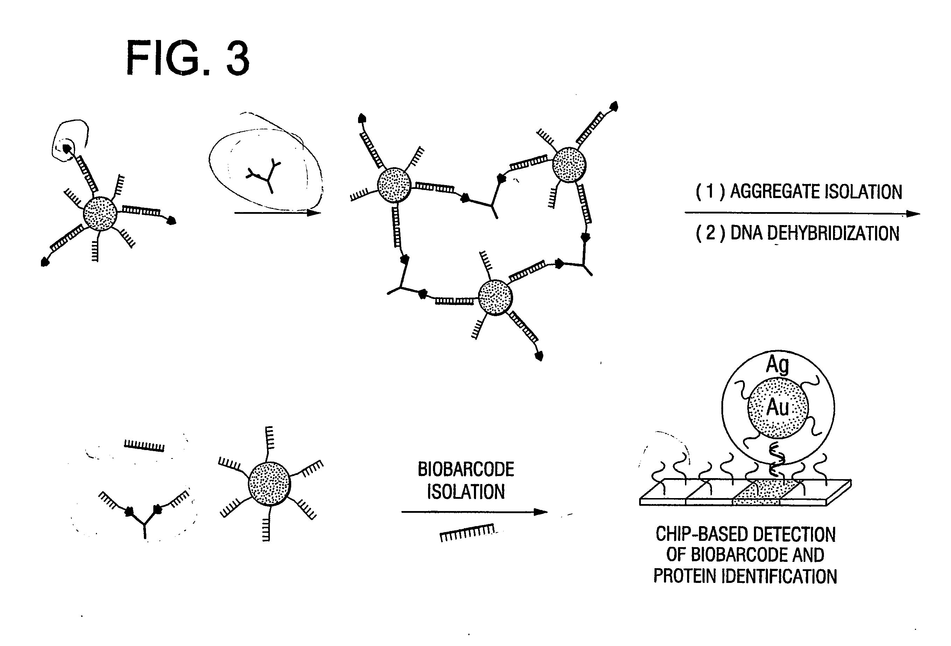

Figure 3 illustrates an array-based protein detection scheme using DNA as a biobarcode for the protein.

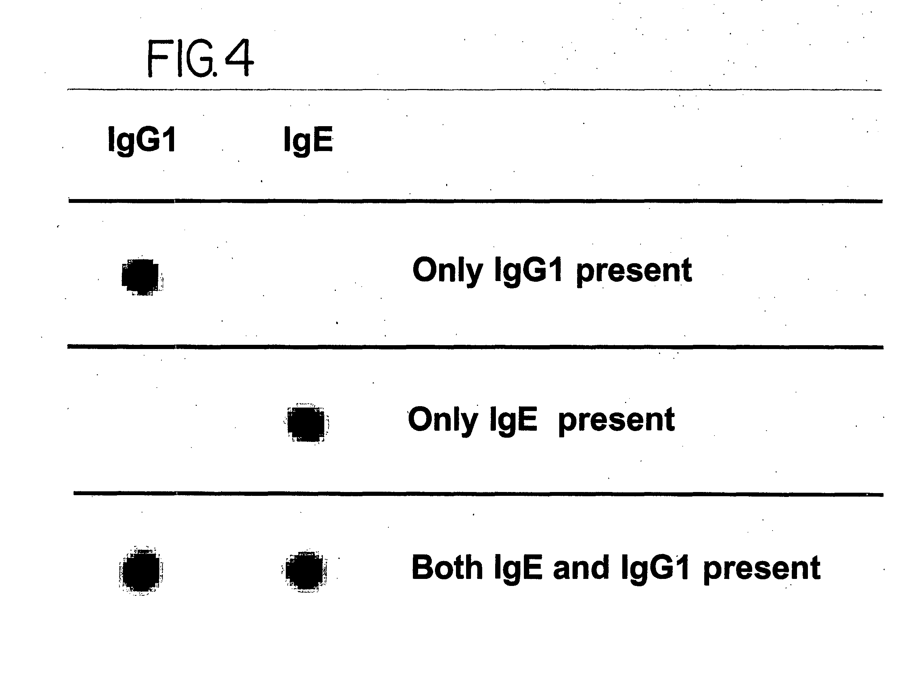

Figure 4 illustrates scanometric DNA array detection of the DNA biobarcodes. Left column is for the detection of the biobarcode associated with IgG1 and the right column is for the biobarcode associated with IgE. The capture oligonucleotides are 5' -thiol-modified ATAACTAGAACTTGA for the IgG1 system and 5' -thiol-modifed TTATCTATTATT for the IgE system. Each spot is approximately 250 um in diameter and read via gray-scale with an Epson Expression 1640XL flatbed scanner (Epson America, Longbeach, California). These assays have been studied and work comparably well over the 20 nM to 700 nM target concentration range.

### DETAILED DESCRIPTION OF THE INVENTION

The present invention relates to a method that utilizes oligonucleotides as biochemical barcodes for detecting multiple protein structures in one solution (Figure 1). The approach takes advantage of protein recognition elements functionalized with oligonucleotide strands and the previous observation that hybridization events that result in the aggregation of gold nanoparticles can significantly alter their physical properties (e.g. optical, electrical, mechanical).⁸⁻¹² The general idea is that each protein recognition element can be encoded with a different oligonucleotide sequence with discrete and tailorable hybridization and melting properties and a physical signature associated with the nanoparticles that changes upon melting to decode a series of analytes in a multi-analyte assay. Therefore, one can use the melting temperature of a DNA-linked aggregate and a physical property associated with the nanoparticles that changes upon melting to decode a series of analytes in a multi-analyte assay. The barcodes herein are different from the ones based on physical diagnostic markers such as nanorods,²³ flourophore-labeled beads,²⁴ and quantum dots,²⁵ in that the decoding information is in the form of chemical information stored in a predesigned oligonucleotide sequence.

In one aspect of the invention, a method for detecting for the presence of a target analyte, e.g., an antibody, in a sample is provided. An antibody such as immunoglobulin E (IgE) or immunoglobulin G1 (IgG1) shown in the Examples below can be detected with olignucleotide-modified probes prehybridized with oligonucleotide strands modified with the appropriate hapten (biotin in the case of IgG1 and dinitrophenyl (DNP) in the case of IgE; Figure 1A). ^{13,14} The DNA sequences in the proof-of-concept assays presented in the Examples below were designed in a way that would ensure that the two different aggregates formed from the probe reactions with IgG1 and IgE would melt at different temperatures, Figure 1B. The probes for IgG1 have longer sequences and greater G,C base contents than those for IgE. Therefore, the former sequences melt at a higher temperature than the latter ones. These sequence variations allow one to prepare probes with distinct melting signatures that can be used as codes to identify which targets have reacted with them to form nanoparticle aggregates. Three different systems have been studied: (1) two probes with one target antibody present (IgG1 or IgE); (2) two probes with the two different target antibodies present, and (3) a control where no target antibodies are present.

In this aspect of the invention, a method is provided for detecting the presence of a target analyte, e.g., an antibody, in a sample comprises contacting a nanoparticle probe having oligonucleotides bound thereto with a sample which may contain a target analyte. At least some of the oligonucleotides attached to the nanoparticle are bound to a first portion of a reporter oligonucleotide as a result of hybridization. A second portion of the reporter oligonucleotide is bound, as a result of hybridization, to an oligonucleotide having bound thereto a specific binding complement (e.g., antigen) to the analyte. The contacting takes place under conditions effective to allow specific binding interactions between the analyte and the nanoparticle probe. In the presence of target analyte, nanoparticle aggregates are produced. These aggregates may be detected by any suitable means.

In practicing the invention, a nanoparticle complex probes are prepared by hybridizing the nanoparticles having oligonucleotides bound thereto with an oligonucleotide modified with a specific binding complement to a target analyte, and a reporter oligonucleotide. At least some of the oligonucleotides attached to the nanoparticle have a sequence that is complementary to a first portion of a reporter oligonucleotide. The oligonucleotides having bound thereto a specific binding complement have a sequence that is complementary to a second portion of a reporter oligonucleotide. The reporter oligonucleotide hybridizes to the at least to some of the oligonucleotides attached to the nanoparticle and to the oligonucleotides having bound thereto the specific binding complement, forming the nanoparticle complex probe under conditions sufficient to allow for hybridization between the components. Any suitable solvent medium and hybridization conditions may be employed in preparing the nanoparticle complex solution that allows for sufficient hybridization of the components. Preferably, the components are hybridized in a phosphate buffered solution (PBS) comprised of 0.3 M NaCl and 10 mM phosphate buffer (pH 7) at room temperature for about 2-3 hours. The concentration of nanoparticle-oligonucleotide conjugates in the hybridization mixture range between about 2 and about 50, preferably about 13 nM. The concentration of hapten-modified oligonucleotides generally ranges between about 50 and about 900, preferably about 300 nM. The concentration of reporter oligonucleotide generally ranges between about 50 and about 900, preferably about 300 nM. Unreacted hapten-modified oligonucleotide and reporter oligonucleotides may be optionally, but preferably, removed by any suitable means, preferably via centrifugation (12,000 rpm, 20 minutes) of the hybridization mixture and subsequent decanting of the supernatant. The prepared complexes were stored in 0.3 M NaCl and 10 mM phosphate buffer (pH 7-7.4), 0.01% azide solution at 4-6 °C.

A typical assay for detecting the presence of a target analyte, e.g, antibody, in a sample is as follows: a solution containing nanoparticle complex probe comprising nanoparticles having oligonucleotides bound thereto, a reporter oligonucleotide, and an oligonucleotide having a specific binding complement to the target analyte, is admixed with an aqueous sample solution believed to contain target protein. The total protein content in the aqueous sample solution generally ranges between about 5 and about 100, usually about 43 ug/ml. The concentration of nanoparticles in the reaction mixture generally ranges between about 2 and about 20, usually about -13 nM. The total volume of the resulting mixture generally ranges between about 100 and about 1000, preferably about 400 uL. Any suitable solvent may be employed in preparing the aqueous sample solution believed to contain target analyte, preferably PBS comprising 0.3 M NaCl and 10 mM phosphate buffer (pH 7-7.4).

The resulting assay mixture is then incubated at a temperature ranging between about 35 and about 40°C, preferably at 37 °C, for a time ranging between about 30 and about 60, preferably about 50 minutes, sufficient to facilitate specific binding pair, e.g., protein-hapten, complexation. If the target protein is present, particle aggregation takes place effecting a shift in the gold nanoparticle plasmon band and a red-to-purple color change along with precipitation. The hybridized products are centrifuged (e.g., 3000 rpm for 2 minutes), and the supernatant containing unreacted elements are decanted prior to analysis.

If desired, the nanoparticle complex probe may be prepared in situ within the assay mixture by admixing all the nanoparticles having oligonucleotides bound thereto, the reporter oligonucleotide, and the hapten-modified oligonucleotide with the sample suspected of containing a target analyte. To ensure complete hybridization among all the components, especially the complementary DNA strands, the assay mixture may be incubated to expedite hybridization at -15 °C for 20 minutes (Boekel Tropicooler Hot/Cold Block Incubator) and stored at 4 °C for 24 hours. In practicing the invention, however, it is preferred that the nanoparticle complex probe is prepared prior to conducting the assay reaction to increase the amount of DNA barcode within the nanoparticle complex probe.

To determine which proteins are present, a melting analysis of the aggregates which monitors the extinction at 260 nm as a function of temperature may carried out in the solution. See, for instance, Figure 2 in Example 3 which describes analysis of a sample containing one or two known target analytes: IgG1 and IgE. As discussed in Example 3, when IgG1 is treated with the probes via the aforementioned protocol, the solution turns pinkish-blue, indicating the formation of nanoparticle aggregates. In a control experiment where no target but background proteins are present, there is no discernible precipitation. A melting analysis of the solution shows a sharp transition with a melting temperature (Tm) of 55 °C. This is the expected transition for the IgG1 target, Figure 2A (---). If IgE is added to a fresh solution of probes, the same color change is observed but the melting analysis provides a curve with a Tm of 36 °C, the expected transition for this target, Figure 2A (-). Significantly, when both protein targets are added to the solution of probes, the solution turns dark purple, and the melting analysis exhibits two distinct transactions. The first derivative of this curve shows two peaks centered at 36 and 55 °C, respectively, Figure 2B. This demonstrates that two distinct assemblies form and their melting properties, which derive from the oligonucleotide barcodes, can be used to distinguish two protein targets.

In another aspect of the invention, a variation of the above aggregation method strategy can be used to increase the sensitivity of the aforementioned system and to increase the number of targets that can be interrogated in one solution. See, for instance, Figure 3 in Example 4. With this strategy, the protein targets can be detected indirectly via the DNA biobarcodes or unique reporter oligonucleotides assigned to specific target analytes. Generally, the suitable length, GC content, and sequence, and selection of the reporter oligonucleotide for the target analyte is predetermined prior to the assay. For instance, a 12-mer oligonucleotide has 4¹² different sequences, many of which can be used to prepare a barcode for a polyvalent protein of interest as shown in Figure 1A. In this variation of the assay, the melting properties of the aggregates that form are not measured in solution but rather the reporter oligonucleotides or DNA biobarcodes within the aggregates are separated via centrifugation (e.g., 3000 rpm for 2 minutes) from the unreacted probes and target molecules. The aggregates are then denatured by any suitable means, e.g., by adding water to the solution, to free the reporter oligonucleotides or biobarcodes. If the reporter oligonucleotide is present in small amounts, it may be amplified by methods known in the art. See, *e*.*g*., Sambrook et al., Molecular Cloning: A Laboratory Manual (2nd ed. 1989) and B.D. Hames and S.J. Higgins, Eds., Gene Probes 1 (IRL Press, New York, 1995). Preferred is polymerase chain reaction (PCR) amplification. The particles and proteins can be separated from the reporter oligonucleotides by any suitable means, e.g., a centrifugal filter device (Millipore Microcon YM-100, 3500 rpm for 25 min. Once the reporter oligonucleotides are isolated, they can be captured on an oligonucleotide array and can be identified using one of the many suitable DNA detection assays (Figure 3). For the examples described herein involving IgG1 and IgE, the reporter oligonucleotides are captured on a microscope slide that has been functionalized with oligonucleotides (250 µm diameter spots) that are complimentary to one half of the barcode of interest (**A3** and **B3** in Figure 1). If the barcode is captured by the oligonucleotide array, a DNA-modified particle that is complementary to the remaining portion of the barcode can be hybridized to the array (see experimental section). When developed via the standard scanometric approach ^{[11]} (which involves treatment with photographic developing solution), a flat bed scanner can be used to quantify the results, Figure 4.¹¹ If IgG1 is present, only the spot designed for IgG1 shows measurable signal. Similarly if IgE is the only protein present, the spot designed for it only exhibits signal. Finally, if both proteins are present, both spots exhibit intense signals.

The present invention is important because it provides two strategies for using nanoparticle probes (preferably gold nanoparticle probes), heavily functionalized with oligonucleotides, to detect single or multiple polyvalent proteins in one solution. Indeed, the detection of multiple proteins in one sample is not trivial and often requires time consuming, expensive assay protocols. In this regard, others have recently used fluorophore-labeled peptidonucleic acids and DNA microarrays to recognize multiple protein targets in one solution. ¹⁵⁻¹⁷ However, this method relies on the binding of the proteins labeled with oligonucleotides to a microarray surface. The final step of the method described herein is based solely on the surface chemistry of ordinary DNA. Therefore, it can incorporate many of the high sensitivity aspects of state-of-the-art nanoparticle DNA detection methods, ^{9,11} but allows one to detect proteins rather than DNA without having the proteins present during the detection event. For surface assays, proteins are typically more difficult to work with than short oligonucleotides because they tend to exhibit greater nonspecific binding to solid supports, which often leads to higher background signals. Finally, for the homogeneous assay, the unusually sharp melting profiles associated with these nanoparticle structures will allow one to design more biobarcodes than what would be possible with probes that exhibit normal and broad DNA melting behavior.

The present invention contemplates the use of any suitable particle having oligonucleotides attached thereto that are suitable for use in detection assays. In practicing this invention, however, nanoparticles are preferred. The size, shape and chemical composition of the particles will contribute to the properties of the resulting probe including the DNA barcode. These properties include optical properties, optoelectronic properties, electrochemical properties, electronic properties, stability in various solutions, pore and channel size variation, ability to separate bioactive molecules while acting as a filter, etc. The use of mixtures of particles having different sizes, shapes and/or chemical compositions, as well as the use of nanoparticles having uniform sizes, shapes and chemical composition, are contemplated. Examples of suitable particles include, without limitation, nano- and microsized core particles, aggregate particles, isotropic (such as spherical particles) and anisotropic particles (such as non-spherical rods, tetrahedral, prisms) and core-shell particles such as the ones described in U.S. Patent application no. 10/034,451, filed December 28, 2002 and International application no. PCT/US01/50825, filed December 28, 2002.

Nanoparticles useful in the practice of the invention include metal (*e*.*g*., gold, silver, copper and platinum), semiconductor (*e*.*g*., CdSe, CdS, and CdS or CdSe coated with ZnS) and magnetic (*e*.*g*., ferromagnetite) colloidal materials. Other nanoparticles useful in the practice of the invention include ZnS, ZnO, TiO₂, AgI, AgBr, HgI₂, PbS, PbSe, ZnTe, CdTe, In₂S₃, In₂Se₃, Cd₃P₂, Cd₃As₂, InAs, and GaAs. The size of the nanoparticles is preferably from about 5 nm to about 150 nm (mean diameter), more preferably from about 5 to about 50 nm, most preferably from about 10 to about 30 nm. The nanoparticles may also be rods, prisms, or tetrahedra.

Methods of making metal, semiconductor and magnetic nanoparticles are well-known in the art. See, *e*.*g*., Schmid, G. (ed.) Clusters and Colloids (VCH, Weinheim, 1994); Hayat, M.A. (ed.) Colloidal Gold: Principles, Methods, and Applications (Academic Press, San Diego, 1991); Massart, R., IEEE Taransactions On Magnetics, 17, 1247 (1981); Ahmadi, T.S. et al., Science, 272, 1924 (1996); Henglein, A. et al., J. Phys. Chem., 99, 14129 (1995); Curtis, A.C., et al., Angew. Chem. Int. Ed. Engl., 27, 1530 (1988).

Methods of making ZnS, ZnO, TiO₂, AgI, AgBr, HgI₂, PbS, PbSe, ZnTe, CdTe, In₂S₃, In₂Se₃, Cd₃P₂, Cd₃As₂, InAs, and GaAs nanoparticles are also known in the art. See, *e*.*g*., Weller, Angew. Chem. Int. Ed. Engl., 32, 41 (1993); Henglein, Top. Curr. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992).

Suitable nanoparticles are also commercially available from, *e*.*g*., Ted Pella, Inc. (gold), Amersham Corporation (gold) and Nanoprobes, Inc. (gold).

Presently preferred for use in detecting nucleic acids are gold nanoparticles. Gold colloidal particles have high extinction coefficients for the bands that give rise to their beautiful colors. These intense colors change with particle size, concentration, interparticle distance, and extent of aggregation and shape (geometry) of the aggregates, making these materials particularly attractive for colorimetric assays. For instance, hybridization of oligonucleotides attached to gold nanoparticles with oligonucleotides and nucleic acids results in an immediate color change visible to the naked eye (see, *e*.*g*., the Examples).

The nanoparticles, the oligonucleotides or both are functionalized in order to attach the oligonucleotides to the nanoparticles. Such methods are known in the art. For instance, oligonucleotides functionalized with alkanethiols at their 3'-termini or 5'-termini readily attach to gold nanoparticles. See Whitesides, Proceedings of the Robert A. Welch Foundation 39th Conference On Chemical Research Nanophase Chemistry, Houston, TX, pages 109-121 (1995). See also, Mucic et al. Chem. Commun. 555-557 (1996) (describes a method of attaching 3' thiol DNA to flat gold surfaces; this method can be used to attach oligonucleotides to nanoparticles). The alkanethiol method can also be used to attach oligonucleotides to other metal, semiconductor and magnetic colloids and to the other nanoparticles listed above. Other functional groups for attaching oligonucleotides to solid surfaces include phosphorothioate groups (see, *e*.*g*., U.S. Patent No. 5,472,881 for the binding of oligonucleotide-phosphorothioates to gold surfaces), substituted alkylsiloxanes (see, *e*.*g*. Burwell, Chemical Technology, 4, 370-377 (1974) and Matteucci and Caruthers, J. Am. Chem. Soc., 103, 3185-3191 (1981) for binding of oligonucleotides to silica and glass surfaces, and Grabar et al., Anal. Chem., 67, 735-743 for binding of aminoalkylsiloxanes and for similar binding of mercaptoaklylsiloxanes). Oligonucleotides terminated with a 5' thionucleoside or a 3' thionucleoside may also be used for attaching oligonucleotides to solid surfaces. The following references describe other methods which may be employed to attached oligonucleotides to nanoparticles: Nuzzo et al., J. Am. Chem. Soc., 109, 2358 (1987) (disulfides on gold); Allara and Nuzzo, Langmuir, 1, 45 (1985) (carboxylic acids on aluminum); Allara and Tompkins, J. Colloid Interface Sci., 49, 410-421 (1974) (carboxylic acids on copper); Iler, The Chemistry Of Silica, Chapter 6, (Wiley 1979) (carboxylic acids on silica); Timmons and Zisman, J. Phys. Chem., 69, 984-990 (1965) (carboxylic acids on platinum); Soriaga and Hubbard, J. Am. Chem. Soc., 104, 3937 (1982) (aromatic ring compounds on platinum); Hubbard, Acc. Chem. Res., 13, 177 (1980) (sulfolanes, sulfoxides and other functionalized solvents on platinum); Hickman et al., J. Am. Chem. Soc., 111, 7271 (1989) (isonitriles on platinum); Maoz and Sagiv, Langmuir, 3, 1045 (1987) (silanes on silica); Maoz and Sagiv, Langmuir, 3, 1034 (1987) (silanes on silica); Wasserman et al., Langmuir, 5, 1074 (1989) (silanes on silica); Eltekova and Eltekov, Langmuir, 3, 951 (1987) (aromatic carboxylic acids, aldehydes, alcohols and methoxy groups on titanium dioxide and silica); Lec et al., J. Phys. Chem., 92, 2597 (1988) (rigid phosphates on metals).

U.S. patent application nos. 09/760,500 and 09/820,279 and international application nos. PCT/US01/01190 and PCT/US01/10071 describe oligonucleotides functionalized with a cyclic disulfide which are useful in practicing this invention. The cyclic disulfides preferably have 5 or 6 atoms in their rings, including the two sulfur atoms. Suitable cyclic disulfides are available commercially or may be synthesized by known procedures. The reduced form of the cyclic disulfides can also be used.

Preferably, the linker further comprises a hydrocarbon moiety attached to the cyclic disulfide. Suitable hydrocarbons are available commercially, and are attached to the cyclic disulfides Preferably the hydrocarbon moiety is a steroid residue. Oligonucleotide-nanoparticle conjugates prepared using linkers comprising a steroid residue attached to a cyclic disulfide have unexpectedly been found to be remarkably stable to thiols (e.g., dithiothreitol used in polymerase chain reaction (PCR) solutions) as compared to conjugates prepared using alkanethiols or acyclic disulfides as the linker. Indeed, the oligonucleotide-nanoparticle conjugates of the invention have been found to be 300 times more stable. This unexpected stability is likely due to the fact that each oligonucleotide is anchored to a nanoparticle through two sulfur atoms, rather than a single sulfur atom. In particular, it is thought that two adjacent sulfur atoms of a cyclic disulfide would have a chelation effect which would be advantageous in stabilizing the oligonucleotide-nanoparticle conjugates. The large hydrophobic steroid residues of the linkers also appear to contribute to the stability of the conjugates by screening the nanoparticles from the approach of water-soluble molecules to the surfaces of the nanoparticles.

In view of the foregoing, the two sulfur atoms of the cyclic disulfide should preferably be close enough together so that both of the sulfur atoms can attach simultaneously to the nanoparticle. Most preferably, the two sulfur atoms are adjacent each other. Also, the hydrocarbon moiety should be large so as to present a large hydrophobic surface screening the surfaces of the nanoparticles.

The oligonucleotide-cyclic nanoparticle conjugates that employ cyclic disulfide linkers may be used as probes in diagnostic assays for detecting target analytes in a sample as described in U.S. patent application nos. 09/760,500 and 09/820,279 and international application nos. PCT/US01/01190 and PCT/US01/10071. These conjugates have been found to improve the sensitivity of diagnostic assays in which they are used. In particular, assays employing oligonucleotide-nanoparticle conjugates prepared using linkers comprising a steroid residue attached to a cyclic disulfide have been found to be about 10 times more sensitive than assays employing conjugates prepared using alkanethiols or acyclic disulfides as the linker.

Each nanoparticle will have a plurality of oligonucleotides attached to it. As a result, each nanoparticle-oligonucleotide conjugate can bind to a plurality of oligonucleotides or nucleic acids having the complementary sequence.

Oligonucleotides of defined sequences are used for a variety of purposes in the practice of the invention. Methods of making oligonucleotides of a predetermined sequence are well-known. See, *e*.*g*., Sambrook et al., Molecular Cloning: A Laboratory Manual (2nd ed. 1989) and F. Eckstein (ed.) Oligonucleotides and Analogues, 1st.Ed. (Oxford University Press, New York, 1991). Solid-phase synthesis methods are preferred for both oligoribonucleotides and oligodeoxyribonucleotides (the well-known methods of synthesizing DNA are also useful for synthesizing RNA). Oligoribonucleotides and oligodeoxyribonucleotides can also be prepared enzymatically. For oligonucleotides having bound thereto a specific binding complement to a target analyte, any suitable method for attaching the specific binding complement such as proteins to the oligonucleotide may be used.

Any suitable method for attaching oligonucleotides onto the nanosphere surface may be used. A particularly preferred method for attaching oligonucleotides onto a surface is based on an aging process described in U.S. application nos. 09/344,667, filed June 25, 1999; 09/603,830, filed June 26, 2000; 09/760,500, filed January 12, 2001; 09/820,279, filed March 28, 2001; 09/927,777, filed August 10, 2001; and in International application nos. PCT/US97/12783, filed July 21, 1997; PCT/US00/17507, filed June 26, 2000; PCT/US01/01190, filed January 12, 2001; PCT/LTS01/10071, filed March 28, 2001. The aging process provides nanoparticle-oligonucleotide conjugates with unexpected enhanced stability and selectivity. The method comprises providing oligonucleotides preferably having covalently bound thereto a moiety comprising a functional group which can bind to the nanoparticles. The moieties and functional groups are those that allow for binding (*i*.*e*., by chemisorption or covalent bonding) of the oligonucleotides to nanoparticles. For instance, oligonucleotides having an alkanethiol, an alkanedisulfide or a cyclic disulfide covalently bound to their 5' or 3' ends can be used to bind the oligonucleotides to a variety of nanoparticles, including gold nanoparticles.

The oligonucleotides are contacted with the nanoparticles in water for a time sufficient to allow at least some of the oligonucleotides to bind to the nanoparticles by means of the functional groups. Such times can be determined empirically. For instance, it has been found that a time of about 12-24 hours gives good results. Other suitable conditions for binding of the oligonucleotides can also be determined empirically. For instance, a concentration of about 10-20 nM nanoparticles and incubation at room temperature gives good results.

Next, at least one salt is added to the water to form a salt solution. The salt can be any suitable water-soluble salt. For instance, the salt may be sodium chloride, magnesium chloride, potassium chloride, ammonium chloride, sodium acetate, ammonium acetate, a combination of two or more of these salts, or one of these salts in phosphate buffer. Preferably, the salt is added as a concentrated solution, but it could be added as a solid. The salt can be added to the water all at one time or the salt is added gradually over time. By "gradually over time" is meant that the salt is added in at least two portions at intervals spaced apart by a period of time. Suitable time intervals can be determined empirically.

The ionic strength of the salt solution must be sufficient to overcome at least partially the electrostatic repulsion of the oligonucleotides from each other and, either the electrostatic attraction of the negatively-charged oligonucleotides for positively-charged nanoparticles, or the electrostatic repulsion of the negatively-charged oligonucleotides from negatively-charged nanoparticles. Gradually reducing the electrostatic attraction and repulsion by adding the salt gradually over time has been found to give the highest surface density of oligonucleotides on the nanoparticles. Suitable ionic strengths can be determined empirically for each salt or combination of salts. A final concentration of sodium chloride of from about 0.1 M to about 1.0 M in phosphate buffer, preferably with the concentration of sodium chloride being increased gradually over time, has been found to give good results.

After adding the salt, the oligonucleotides and nanoparticles are incubated in the salt solution for an additional period of time sufficient to allow sufficient additional oligonucleotides to bind to the nanoparticles to produce the stable nanoparticle-oligonucleotide conjugates. As will be described in detail below, an increased surface density of the oligonucleotides on the nanoparticles has been found to stabilize the conjugates. The time of this incubation can be determined empirically. A total incubation time of about 24-48, preferably 40 hours, has been found to give good results (this is the total time of incubation; as noted above, the salt concentration can be increased gradually over this total time). This second period of incubation in the salt solution is referred to herein as the "aging" step. Other suitable conditions for this "aging" step can also be determined empirically. For instance, incubation at room temperature and pH 7.0 gives good results.

The conjugates produced by use of the "aging" step have been found to be considerably more stable than those produced without the "aging" step. As noted above, this increased stability is due to the increased density of the oligonucleotides on the surfaces of the nanoparticles which is achieved by the "aging" step. The surface density achieved by the "aging" step will depend on the size and type of nanoparticles and on the length, sequence and concentration of the oligonucleotides. A surface density adequate to make the nanoparticles stable and the conditions necessary to obtain it for a desired combination of nanoparticles and oligonucleotides can be determined empirically. Generally, a surface density of at least 10 picomoles/cm² will be adequate to provide stable nanoparticle-oligonucleotide conjugates. Preferably, the surface density is at least 15 picomoles/cm². Since the ability of the oligonucleotides of the conjugates to hybridize with nucleic acid and oligonucleotide targets can be diminished if the surface density is too great, the surface density is preferably no greater than about 35-40 picomoles/cm².

As used herein, "stable" means that, for a period of at least six months after the conjugates are made, a majority of the oligonucleotides remain attached to the nanoparticles and the oligonucleotides are able to hybridize with nucleic acid and oligonucleotide targets under standard conditions encountered in methods of detecting nucleic acid and methods of nanofabrication.

It has been found that the hybridization efficiency of nanoparticle-oligonucleotide conjugates can be increased dramatically by the use of recognition oligonucleotides which comprise a recognition portion and a spacer portion. "Recognition oligonucleotides" are oligonucleotides which comprise a sequence complementary to at least a portion of the sequence of a nucleic acid or oligonucleotide target. In this embodiment, the recognition oligonucleotides comprise a recognition portion and a spacer portion, and it is the recognition portion which hybridizes to the nucleic acid or oligonucleotide target. The spacer portion of the recognition oligonucleotide is designed so that it can bind to the nanoparticles. For instance, the spacer portion could have a moiety covalently bound to it, the moiety comprising a functional group which can bind to the nanoparticles. These are the same moieties and functional groups as described above. As a result of the binding of the spacer portion of the recognition oligonucleotide to the nanoparticles, the recognition portion is spaced away from the surface of the nanoparticles and is more accessible for hybridization with its target. The length and sequence of the spacer portion providing good spacing of the recognition portion away from the nanoparticles can be determined empirically. It has been found that a spacer portion comprising at least about 10 nucleotides, preferably 10-30 nucleotides, gives good results. The spacer portion may have any sequence which does not interfere with the ability of the recognition oligonucleotides to become bound to the nanoparticles or to a nucleic acid or oligonucleotide target. For instance, the spacer portions should not sequences complementary to each other, to that of the recognition olignucleotides, or to that of the nucleic acid or oligonucleotide target of the recognition oligonucleotides. Preferably, the bases of the nucleotides of the spacer portion are all adenines, all thymines, all cytidines, or all guanines, unless this would cause one of the problems just mentioned. More preferably, the bases are all adenines or all thymines. Most preferably the bases are all thymines.

It has further been found that the use of diluent oligonucleotides in addition to recognition oligonucleotides provides a means of tailoring the conjugates to give a desired level of hybridization. The diluent and recognition oligonucleotides have been found to attach to the nanoparticles in about the same proportion as their ratio in the solution contacted with the nanoparticles to prepare the conjugates. Thus, the ratio of the diluent to recognition oligonucleotides bound to the nanoparticles can be controlled so that the conjugates will participate in a desired number of hybridization events. The diluent oligonucleotides may have any sequence which does not interfere with the ability of the recognition oligonucleotides to be bound to the nanoparticles or to bind to a nucleic acid or oligonucleotide target. For instance, the diluent oligonulceotides should not have a sequence complementary to that of the recognition olignucleotides or to that of the nucleic acid or oligonucleotide target of the recognition oligonucleotides. The diluent oligonucleotides are also preferably of a length shorter than that of the recognition oligonucleotides so that the recognition oligonucleotides can bind to their nucleic acid or oligonucleotide targets. If the recognition oligonucleotides comprise spacer portions, the diluent oligonulceotides are, most preferably, about the same length as the spacer portions. In this manner, the diluent oligonucleotides do not interefere with the ability of the recognition portions of the recognition oligonucleotides to hybridize with nucleic acid or oligonucleotide targets. Even more preferably, the diluent oligonucleotides have the same sequence as the sequence of the spacer portions of the recognition oligonucleotides.

For detection of the presence of a target analyte in a sample, particle complex probes, preferably nanoparticle complex probes, are used. These particle complexes may be generated prior to conducting the actual assay or in situ while conducting the assay. These complexes comprise a particle, preferably a nanoparticle, having oligonucleotides bound thereto, a reporter oligonucleotide, and an oligonucleotide having bound thereto a specific binding complement of a target analyte. The DNA barcode or reporter oligonucleotides has a sequence having at least two portions and joins via hybridization the nanoparticle having oligonucleotides bound thereto and the oligonucleotide having bound thereto the specific binding complement. The oligonucleotides bound to the nanoparticles have a sequence that is complementary to one portion of the reporter oligonucleotide and the oligonucleotide having bound thereto the specific binding complement having a sequence that is complementary to a second portion of the reporter oligonucleotide. The reporter oligonucleotides have at least two portions and joins via hybridization the nanoparticle having oligonucleotides bound thereto and the oligonucleotide having bound thereto the specific binding complement. When employed in a sample containing the target analyte, the nanoparticle complex binds to the target analyte and aggregation occurs. The aggregates may be isolated and subject to further melting analysis to identify the particular target analyte where multiple targets are present as discussed above. Alternatively, the aggregates can be dehybridized to release the reporter oligonucleotides. These reporter oligonucleotides can then be detected by any suitable DNA detection system using any suitable detection probes.

In another aspect of the invention, the reporter oligonucleotides released by dehybridization of the aggregates can be detected using a substrate having oligonucleotides bound thereto. The oligonucleotides have a sequence complementary to at least one portion of the reporter oligonucleotides. Some embodiments of the method of detecting the reporter oligonucleotides utilize a substrate having complementary oligonucleotides bound thereto to capture the reporter oligonucleotides. These captured reporter oligonucleotides are then detected by any suitable means. By employing a substrate, the detectable change (the signal) can be amplified and the sensitivity of the assay increased.

Any substrate can be used which allows observation of the detectable change. Suitable substrates include transparent solid surfaces (*e*.*g*., glass, quartz, plastics and other polymers), opaque solid surface (*e*.*g*., white solid surfaces, such as TLC silica plates, filter paper, glass fiber filters, cellulose nitrate membranes, nylon membranes), and conducting solid surfaces (*e*.*g*., indium-tin-oxide (ITO)). The substrate can be any shape or thickness, but generally will be flat and thin. Preferred are transparent substrates such as glass (*e*.*g*., glass slides) or plastics (*e*.*g*., wells of microtiter plates).

Any suitable method for attaching oligonucleotides to a substrate may be used. For instance, oligonucleotides can be attached to the substrates as described in, e.g., Chrisey et al., Nucleic Acids Res., 24, 3031-3039 (1996); Chrisey et al., Nucleic Acids Res., 24, 3040-3047 (1996); Mucic et al., Chem. Commun., 555 (1996); Zimmermann and Cox, Nucleic Acids Res., 22, 492 (1994); Bottomley et al., J. Vac. Sci. Technol. A, 10, 591 (1992); and Hegner et al., FEBS Lett., 336, 452 (1993).

The oligonucleotides attached to the substrate have a sequence complementary to a first portion of the sequence of reporter oligonucleotides to be detected. The reporter oligonucleotide is contacted with the substrate under conditions effective to allow hybridization of the oligonucleotides on the substrate with the reporter oligonucleotide. In this manner the reporter oligonucleotide becomes bound to the substrate. Any unbound reporter oligonucleotide is preferably washed from the substrate before adding a detection probe such as nanoparticle-oligonucleotide conjugates.

In one aspect of the invention, the reporter oligonucleotide bound to the oligonucleotides on the substrate is contacted with a first type of nanoparticles having oligonucleotides attached thereto. The oligonucleotides have a sequence complementary to a second portion of the sequence of the reporter oligonucleotide, and the contacting takes place under conditions effective to allow hybridization of the oligonucleotides on the nanoparticles with the reporter oligonucleotide. In this manner the first type of nanoparticles become bound to the substrate. After the nanoparticle-oligonucleotide conjugates are bound to the substrate, the substrate is washed to remove any unbound nanoparticle-oligonucleotide conjugates.

The oligonucleotides on the first type of nanoparticles may all have the same sequence or may have different sequences that hybridize with different portions of the reporter oligonucleotide to be detected. When oligonucleotides having different sequences are used, each nanoparticle may have all of the different oligonucleotides attached to it or, preferably, the different oligonucleotides are attached to different nanoparticles. Alternatively, the oligonucleotides on each of the first type of nanoparticles may have a plurality of different sequences, at least one of which must hybridize with a portion of the reporter oligonucleotide to be detected.

Optionally, the first type of nanoparticle-oligonucleotide conjugates bound to the substrate is contacted with a second type of nanoparticles having oligonucleotides attached thereto. These oligonucleotides have a sequence complementary to at least a portion of the sequence(s) of the oligonucleotides attached to the first type of nanoparticles, and the contacting takes place under conditions effective to allow hybridization of the oligonucleotides on the first type of nanoparticles with those on the second type of nanoparticles. After the nanoparticles are bound, the substrate is preferably washed to remove any unbound nanoparticle-oligonucleotide conjugates.

The combination of hybridizations produces a detectable change. The detectable changes are the same as those described above, except that the multiple hybridizations result in an amplification of the detectable change. In particular, since each of the first type of nanoparticles has multiple oligonucleotides (having the same or different sequences) attached to it, each of the first type of nanoparticle-oligonucleotide conjugates can hybridize to a plurality of the second type of nanoparticle-oligonucleotide conjugates. Also, the first type of nanoparticle-oligonucleotide conjugates may be hybridized to more than one portion of the reporter oligonucleotide to be detected. The amplification provided by the multiple hybridizations may make the change detectable for the first time or may increase the magnitude of the detectable change. This amplification increases the sensitivity of the assay, allowing for detection of small amounts of reporter oligonucleotide.

If desired, additional layers of nanoparticles can be built up by successive additions of the first and second types of nanoparticle-oligonucleotide conjugates. In this way, the number of nanoparticles immobilized per molecule of target nucleic acid can be further increased with a corresponding increase in intensity of the signal.

Also, instead of using first and second types of nanoparticle-ofigonucleotide conjugates designed to hybridize to each other directly, nanoparticles bearing oligonucleotides that would serve to bind the nanoparticles together as a consequence of hybridization with binding oligonucleotides could be used.

When a substrate is employed, a plurality of the initial types of nanoparticle-oligonucleotide conjugates or oligonucleotides can be attached to the substrate in an array for detecting multiple portions of a target reporter oligonucleotide, for detecting multiple different reporter oligonucleotides, or both. For instance, a substrate may be provided with rows of spots, each spot containing a different type of oligonucleotide designed to bind to a portion of a target reporter oligonucleotide. A sample containing one or more reporter oligonucleotides is applied to each spot, and the rest of the assay is performed in one of the ways described above using appropriate oligonucleotide-nanoparticle conjugates.

Finally, when a substrate is employed, a detectable change can be produced or further enhanced by silver staining. Silver staining can be employed with any type of nanoparticles that catalyze the reduction of silver. Preferred are nanoparticles made of noble metals (*e*.*g*., gold and silver). See Bassell, et al., J. Cell Biol., 126, 863-876 (1994); Braun-Howland et al., Biotechniques, 13, 928-931 (1992). If the nanoparticles being employed for the detection of a nucleic acid do not catalyze the reduction of silver, then silver ions can be complexed to the nucleic acid to catalyze the reduction. See Braun et al., Nature, 391, 775 (1998). Also, silver stains are known which can react with the phosphate groups on nucleic acids.

Silver staining can be used to produce or enhance a detectable change in any assay performed on a substrate, including those described above. In particular, silver staining has been found to provide a huge increase in sensitivity for assays employing a single type of nanoparticle so that the use of layers of nanoparticles can often be eliminated.

In assays for detecting reporter oligonucleotides performed on a substrate, the detectable change can be observed with an optical scanner. Suitable scanners include those used to scan documents into a computer which are capable of operating in the reflective mode (*e*.*g*., a flatbed scanner), other devices capable of performing this function or which utilize the same type of optics, any type of greyscale-sensitive measurement device, and standard scanners which have been modified to scan substrates according to the invention (*e*.*g*., a flatbed scanner modified to include a holder for the substrate) (to date, it has not been found possible to use scanners operating in the transmissive mode). The resolution of the scanner must be sufficient so that the reaction area on the substrate is larger than a single pixel of the scanner. The scanner can be used with any substrate, provided that the detectable change produced by the assay can be observed against the substrate (*e*.*g*., a grey spot, such as that produced by silver staining, can be observed against a white background, but cannot be observed against a grey background). The scanner can be a black-and-white scanner or, preferably, a color scanner. Most preferably, the scanner is a standard color scanner of the type used to scan documents into computers. Such scanners are inexpensive and readily available commercially. For instance, an Epson Expression 636 (600 x 600 dpi), a UMAX Astra 1200 (300 x 300 dpi), or a Microtec 1600 (1600 x 1600 dpi) can be used. The scanner is linked to a computer loaded with software for processing the images obtained by scanning the substrate. The software can be standard software which is readily available commercially, such as Adobe Photoshop 5.2 and Corel Photopaint 8.0. Using the software to calculate greyscale measurements provides a means of quantitating the results of the assays. The software can also provide a color number for colored spots and can generate images (*e*.*g*., printouts) of the scans which can be reviewed to provide a qualitative determination of the presence of a nucleic acid, the quantity of a nucleic acid, or both. The computer can be a standard personal computer which is readily available commercially. Thus, the use of a standard scanner linked to a standard computer loaded with standard software can provide a convenient, easy, inexpensive means of detecting and quantitating nucleic acids when the assays are performed on substrates. The scans can also be stored in the computer to maintain a record of the results for further reference or use. Of course, more sophisticated instruments and software can be used, if desired.

A universal nanoparticle-oligonucleotide conjugate which may be used in an assay for any target reporter oligonucleotide. This "universal probe" has oligonucleotides of a single sequence attached to it and is complementary with a portion of the reporter oligonucleotide. These oligonucleotides bound to the universal probe can hybridize with a portion of the reporter oligonucleotides bound to the support. The first portion is complementary to at least a portion of the sequence of the oligonucleotides on the nanoparticles. The second portion is complementary to a portion of the sequence of the nucleic acid to be detected. A plurality of binding oligonucleotides having the same first portion and different second portions can be used, in which case the "universal probe", after hybridization to the binding oligonucleotides, can bind to multiple portions of the nucleic acid to be detected or to different nucleic acid targets.

It is to be noted that the term "a" or "an" entity refers to one or more of that entity. For example, "a characteristic" refers to one or more characteristics or at least one characteristic. As such, the terms "a" (or "an"), "one or more" and "at least one" are used interchangeably herein. It is also to be noted that the terms "comprising", "including", and "having" have been used interchangeably.

### EXAMPLES

### Example 1: Preparation of Oligonucleotide-Modified Gold Nanoparticles A. Preparation Of Gold Nanoparticles

Oligonucleotide-modified 13 nm Au particles were prepared by literature methods (∼110 oligonucleotides/particle)¹⁸⁻²⁰. Gold colloids (13 nm diameter) were prepared by reduction of HAuCl₄ with citrate as described in Frens, Nature Phys. Sci., 241, 20 (1973) and Grabar, Anal. Chem., 67, 735 (1995). Briefly, all glassware was cleaned in aqua regia (3 parts HCl, 1 part HNO₃), rinsed with Nanopure H₂O, then oven dried prior to use. HAuCl₄ and sodium citrate were purchased from Aldrich Chemical Company. An aqueous solution of HAuCl₄ (1 mM, 500 mL) was brought to a reflux while stirring, and then 50 mL of a 38.8 mM trisodium citrate solution was added quickly, which resulted in a change in solution color from pale yellow to deep red. After the color change, the solution was refluxed for an additional fifteen minutes, allowed to cool to room temperature, and subsequently filtered through a Micron Separations Inc. 0.45 micron nylon filter. Au colloids were characterized by UV-vis spectroscopy using a Hewlett Packard 8452A diode array spectrophotometer and by Transmission Electron Microscopy (TEM) using a Hitachi 8100 transmission electron microscope. A typical solution of 13 nm diameter gold particles exhibited a characteristic surface plasmon band centered at 518 - 520 nm. Gold particles with diameters of 13 nm will produce a visible color change when aggregated with target and probe oligonucleotide sequences in the 10-72 nucleotide range.

### B. Synthesis Of Oligonucleotides

Oligonucleotides were synthesized on a 1 micromole scale using a Milligene Expedite DNA synthesizer in single column mode using phosphoramidite chemistry. Eckstein, F. (ed.) Oligonucleotides and Analogues: A Practical Approach (IRL Press, Oxford, 1991). All solutions were purchased from Milligene (DNA synthesis grade). Average coupling efficiency varied from 98 to 99.8%, and the final dimethoxytrityl (DMT) protecting group was not cleaved from the oligonucleotides to aid in purification.

For 3'-thiol-oligonucleotides, Thiol-Modifier C3 S-S CPG support was purchased from Glen Research and used in the automated synthesizer. The final dimethoxytrityl (DMT) protecting group was not removed to aid in purification. After synthesis, the supported oligonucleotide was placed in 1 mL of concentrated ammonium hydroxide for 16 hours at 55 °C to cleave the oligonucleotide from the solid support and remove the protecting groups from the bases.

After evaporation of the ammonia, the oligonucleotides were purified by preparative reverse-phase HPLC using an HP ODS Hypersil column (5 µm, 250 x 4 mm) with 0.03 M triethyl ammonium acetate (TEAA), pH 7 and a 1%/minute gradient of 95% CH₃CN/5% 0.03 M TEAA at a flow rate of 1 mL/minute, while monitoring the UV signal of DNA at 254 nm. The retention time of the DMT protected modified 12-base oligomer was 30 minutes. The DMT was subsequently cleaved by soaking the purified oligonucleotide in an 80 % acetic acid solution for 30 minutes, followed by evaporation; the oligonucleotide was redispersed in 500 µL of water, and the solution was extracted with ethyl acetate (3 x 300 µL). After evaporation of the solvent, the oligonucleotide(10 OD's) was redispersed in 100 υL of a 0.04 M DTT, 0.17 M phosphate buffer (pH 8) solution overnight at 50°C to cleave the 3' disulfide. Aliquots of this solution (< 10 OD's) were purified through a desalting NAP-5 column. The amount of oligonucleotide was determined by absorbance at 260 nm. Purity was assessed by ion-exchange HPLC using a Dionex Nucleopac PA-100 column (250 x 4 mm) with 10 mM NaOH (pH 12) and a 2%/minute gradient of 10 mM NaOH, 1 M NaCl at a flow rate of 1 mL/minute while monitoring the UV signal of DNA at 254 nm. Three peaks with retention times (Tᵣ) of 18.5, 18.9 and 22 minutes were observed. The main single peak at Tᵣ = 22.0 minutes, which has been attributed to the disulfide, was 79 % by area. The two peaks with shorter retention times of 18.5 and 18.9 minutes were ∼9 % and 12 % by area respectively, and have been attributed to oxidized impurity and residual thiol oligonucleotide.

5'-Alkylthiol modified oligonucleotides were prepared using the following protocol: 1) a CPG-bound, detritylated oligonucleotide was synthesized on an automated DNA synthesizer (Expedite) using standard procedures; 2) the CPG-cartridge was removed and disposable syringes were attached to the ends; 3) 200 µL of a solution containing 20 µmole of 5-Thiol-Modifier C6-phosphoramidite (Glen Research) in dry acetonitrile was mixed with 200 µL of standard "tetrazole activator solution" and, *via* one of the syringes, introduced into the cartridge containing the oligonucleotide-CPG; 4) the solution was slowly pumped back and forth through the cartridge for 10 minutes and then ejected followed by washing with dry acetonitrile (2 x 1 mL); 5) the intermediate phosphite was oxidized with 700 µL of 0.02 M iodine in THF/pyridine/water (30 seconds) followed by washing with acetonitrile/pyridine (1:1; 2 x 1 mL) and dry acetonitirile. The trityloligonucleotide derivative was then isolated and purified as described by the 3'-alkylthiol oligonucleotides; then the trityl protecting group was cleaved by adding 15 uL (for 10 OD's) of a 50 mM AgNO₃ solution to the dry oligonucleotide sample for 20 minutes, which resulted in a milky white suspension. The excess silver nitrate was removed by adding 20 ΦL of a 10 mg/mL solution of DTT (five minute reaction time), which immediately formed a yellow precipitate that was removed by centrifugation. Aliquots of the oligonucleotide solution (<10 OD's) were then transferred onto a desalting NAP-5 column for purification. The final amount and the purity of the resulting 5' alkylthiol oligonucleotides were assessed using the techniques described above for 3' alkylthiol oligonucleotides. Two major peaks were observed by ion-exchange HPLC with retention times of 19.8 minutes (thiol peak, 16 % by area) and 23.5 minutes (disulfide peak, 82 % by area).

### C. Attachment Of Oligonucleotides To Gold Nanoparticles

A 1 mL solution of the gold colloids (17nM) in water was mixed with excess (3.68 υM) thiol-oligonucleotide (22 bases in length) in water, and the mixture was allowed to stand for 12-24 hours at room temperature. Then, the solution was brought to 0.1 M NaCl, 10 mM phosphate buffer (pH 7) and allowed to stand for 40 hours. This "aging" step was designed to increase the surface coverage by the thiol-oligonucleotides and to displace oligonucleotide bases from the gold surface. The solution was next centrifuged at 14,000 rpm in an Eppendorf Centrifuge 5414 for about 25 minutes to give a very pale pink supernatant containing most of the oligonucleotide (as indicated by the absorbance at 260 nm) along with 7-10% of the colloidal gold (as indicated by the absorbance at 520 nm), and a compact, dark, gelatinous residue at the bottom of the tube. The supernatant was removed, and the residue was resuspended in about 200 µL of buffer (10 mM phosphate, 0.1 M NaCl) and recentrifuged. After removal of the supernatant solution, the residue was taken up in 1.0 mL of buffer (10 mM phosphate, 0.3 M NaCl, 0.01% NaN₃). The resulting red master solution was stable (*i*.*e*., remained red and did not aggregate) on standing for months at room temperature, on spotting on silica thin-layer chromatography (TLC) plates (see Example 4), and on addition to 1 M NaCl, 10 mM MgCl₂, or solutions containing high concentrations of salmon sperm DNA.

### Example 2: Preparation of Hapten-modified oligonucleotides

Hapten-modified oligonucleotides were prepared with a biotin-triethylene glycol phosphoramidite for **A1** and 2, 4-dinitrophenyl-triethylene glycol phosphoramidite for **B1** (Glen research) using standard solid-phase DNA synthesis procedures.²¹

Biotin modified oligonucleotides were prepared using the following protocol: A CPG-bound, detritylated oligonucleotide was synthesized on an automated DNA synthesizer (Expedite) using standard procedures²¹. The CPG-cartridge was then removed and disposable syringes were attached to the ends. 200 µL of a solution containing 20 µmole of biotin-triethylene glycol phosphoramidite in dry acetonitrile was then mixed with 200 µL of standard "tetrazole activator solution" and, *via* one of the syringes, introduced into the cartridge containing the oligonucleotide-CPG. The solution then was slowly pumped back and forth through the cartridge for 10 minutes and then ejected followed by washing with dry acetonitrile (2 x 1 mL). Thereafter, the intermediate phosphite was oxidized with 700 µL of 0.02 M iodine in THF/pyridine/water (30 seconds) followed by washing with acetonitrile/pyridine (1:1; 2 x 1 mL) and dry acetonitirile with subsequent drying of the column with a stream of nitrogen. The trityl protecting group was not removed, which aids in purification. The supported oligonucleotide was placed in 1 mL of concentrated ammonium hydroxide for 16 hours at 55 °C to cleave the oligonucleotide from the solid support and remove the protecting groups from the bases. After evaporation of the ammonia, the oligonucleotides were purified by preparative reverse-phase HPLC using an HP ODS Hypersil column (5 µm, 250 x 4 mm) with 0.03 M triethyl ammonium acetate (TEAA), pH 7 and a 1%/minute gradient of 95% CH₃CN/5% 0.03 M TEAA at a flow rate of 1 mL/minute, while monitoring the UV signal of DNA at 254 nm. The retention time of the DMT protected oligonucleotides was ∼32 minutes. The DMT was subsequently cleaved by soaking the purified oligonucleotide in an 80 % acetic acid solution for 30 minutes, followed by evaporation; the oligonucleotide was redispersed in 500 µL of water, and the solution was extracted with ethyl acetate (3 x 300 µL) and dried. The same protocol was used to synthesize DNP modified oligonucleotide using 2, 4-dinitrophenyl-triethylene glycol phosphoramidite.

### Example 3: Assay Using Nanoparticle Complex Probes

The Oligonucleotide-modified 13 nm gold particles were prepared as described in Example 1. Hapten-modified oligonucleotides were prepared as described in Example 2 with a biotin-triethylene glycol phosphoramidite for **A1** and 2,4-dinitrophenyl-triethylene glycol phosphoramidite for **B1** (Glen research) using standard solid-phase DNA synthesis procedures.²¹ The PBS buffer solution used in this research consists of 0.3 M NaCl and 10 mM phosphate buffer (pH 7). IgE and IgG1 were purchased from Sigma Aldrich (Milwaukee, WI) and dissolved in 0.3 M PBS buffer with 0.05% Tween 20 (final concentration: 4.3x10⁻⁸ b/µl) and background proteins (10 ug/ml of lysozyme, 1% bovine serum albumin, and 5.3 ug/ml of anti-digoxin; 10 uL of each) prior to use.

To prepare the probes, the oligonucleotide modified particles (13 nM, 300 µL) were hybridized with hapten-modified complementary oligonucleotides (10 µL of 10 µM) and biobarcode DNA (10 µL of 10 µM) at room temperature for 2-3 h, sequences given in Figure 1. Unreacted hapten-modified oligonucleotide and biobarcodes were removed via centrifugation (12,000 rpm, 20 min) of the nanoparticle probes and subsequent decanting of the supernatant.

In a typical assay for IgE and/or IgG1, the target proteins (40 µl of 43 µg/ml for each) were added to the solution containing the probes (∼13 nM), and the mixture was incubated at 37 °C for 50 minutes to facilitate protein-hapten complexation. To ensure complete reaction among all the components, especially the complementary DNA strands, the solution was incubated to expedite hybridization at -15 °C for 20 minutes (Boekel Tropicooler Hot/Cold Block Incubator) and stored at 4 °C for 24 hours. If the target protein is present, particle aggregation takes place effecting a shift in the gold nanoparticle plasmon band and a red-to-purple color change along with precipitation. The hybridized products were centrifuged (3000 rpm for 2 minutes), and the supernatant containing unreacted elements was decanted prior to analysis. To determine which proteins are present, a melting analysis which monitors the extinction at 260 nm as a function of temperature is carried out in the solution, Figure 2. When IgG1 is treated with the probes via the aforementioned protocol, the solution turns pinkish-blue, indicating the formation of nanoparticle aggregates. In a control experiment where no target but background proteins are present, there is no discernible precipitation. A melting analysis of the solution shows a sharp transition with a melting temperature (Tm) of 55 °C. This is the expected transition for the IgG1 target, Figure 2A (---). If IgE is added to a fresh solution of probes, the same color change is observed but the melting analysis provides a curve with a Tm of 36 °C, the expected transition for this target, Figure 2A (-). Significantly, when both protein targets are added to the solution of probes, the solution turns dark purple, and the melting analysis exhibits two distinct transactions. The first derivative of this curve shows two peaks centered at 36 and 55 °C, respectively, Figure 2B. This demonstrates that two distinct assemblies form and their melting properties, which derive from the oligonucleotide barcodes, can be used to distinguish two protein targets.

### Example 4: Assay Using Nanoparticle Complex Probes

A variation of this strategy can be used to increase the sensitivity of the aforementioned system and to increase the number of targets that can be interrogated in one solution (Figure 3). With this strategy, the protein targets can be detected indirectly via the DNA biobarcodes. A 12-mer oligonucleotide has 4¹² different sequences, many of which can be used to prepare a barcode for a polyvalent protein of interest via Figure 1A. In this variation of the assay, the melting properties of the aggregates that form are not measured in solution but rather the DNA biobarcodes within the aggregates are separated via centrifugation (3000 rpm fro 2 minutes) from the unreacted probes and target molecules. The aggregates are then denatured by adding water to the solution, freeing the complexed DNA. The particles and proteins can be separated from the DNA barcodes with a centrifugal filter device (Millipore Microcon YM-100, 3500 rpm for 25 min). Once the DNA barcodes are isolated, they can be captured on an oligonucleotide array and can be identified using one of the many DNA detection assays (Figure 3). For the examples described herein involving IgG1 and IgE, the barcodes are captured on a microscope slide that has been functionalized with oligonucleotides (250 µm diameter spots) that are complementary to one half of the barcode of interest (**A3** and **B3** in Figure 1). If the barcode is captured by the oligonucleotide array, a DNA-modified particle that is complementary to the remaining portion of the barcode can be hybridized to the array (see experimental section). When developed via the standard scanometric approach ^{[11]} (which involves treatment with photographic developing solution), a flat bed scanner can be used to quantify the results, Figure 4.¹¹ If IgG1 is present, only the spot designed for IgG1 shows measurable signal. Similarly if IgE is the only protein present, the spot designed for it only exhibits signal. Finally, if both proteins are present, both spots exhibit intense signals.

For scanometric DNA biobarcode detection, the DNA/Au nanoparticle assembly was centrifuged (3000 rpm for 2 min) in a polystyrene 1.5 mL vial, and the supernatant was removed. PBS buffer solution (700 µl) was added to the aggregate and the procedure was repeated to ensure isolation of the aggregate from unreacted protein and assay components. Then, 500 µl of water was added to the vial containing the aggregate to denature it. Microarrays were prepared and DNA hybridization methods were used according to literature methods.^{11,22} The isolated DNA biobarcodes were premixed with **A2**-modified nanoparticles or **B2**-modified nanoparticles (2nM), exposed to the DNA microarray, and incubated in a hybridization chamber (GRACE BIO-LABS) at room temperature for three hours. The array was then washed with 0.3M NaNO₃ and 10nM NaH₂PO₄/Na₂HPO₄ buffer (pH 7) and submerged in Silver Enhancer Solution (Sigma) for three minutes at room temperature. The slide was washed with water and then analyzed with a flat bed scanner.

### References

(1) A. Pandey and M. Mann, Nature 2000, 405, 837-846.
(2) S. Fields and O. K. Song, Nature 1989, 340, 245-246.
(3) M. Ijksma, B. Kamp, J. C. Hoogvliet, W. P. van Bennekom, Anal. Chem. 2001, 73, 901-907.
(4) R. F. Service, Science, 2000,287,2136-2138.
(5) H. Zole, Monoclonal Antibodies, Springer-Verlag, New York, 2000, p.1-5.
(6) J. E. Butler, J. Immunoassay, 2000, 21(2 & 3), 165-209.
(7) P. Herbrink, A. Noorduyn, W. C. Van Dijk, Tech. Diagn. Pathol. 1991, 2, 1-19.
(8) C. A. Mirkin, R. L. Letsinger, R. C. Mucic, J. J. Storhoff, Nature 1996, 382, 607-609
(9) J. J. Storhoff, C. A. Mirkin, Chem. Rev. 1999, 99, 1849-1862.
(10) S. -J. Park, A. A. Lazarides, C. A. Mirkin, P. W. Brazis, C. R. Kannewurf, R. L. Letsinger, Angew. Chem. Int. Ed. 2000, 39, 3845-3848.
(11) T. A. Taton, C. A. Mirkin, and R. L. Letsinger, Science 2000, 289, 1757-1760.
(12) S. -J. Park, A. A. Lazarides, C. A. Mirkin, and R. L. Letsinger, Angew. Chem. Int. Ed. 2001, 40, 2909-2912.
(13) Z. Eshhar, M. Ofarim, and T. Waks, J. Immunol. 1980, 124, 775-780.
(14) M. Wilcheck and E. A. Bayer, Immunol. Today 1984, 5, 39-43
(15) N: Winssinger, J.L. Harris, B.J. Backes, P.G. Schultz, Agnew. Chem. Int. Ed. 2001, 40,3152-3155
(16) G. MacBeath, A.N. Koehler, S.L. Schreiber, J. Am. Chem. Soc. 1999, 121, 7967-7968.
(17) P. J. Hergenrother, K.M. Depew, S.L. Schreiber, J. Am. Chem. Soc. 2000, 122, 7849-7850.
(18) J. J. Storhoff, R. Elghanian, R.C. Mucic, C.A. Mirkin, R.L. Letsinger, J. Am. Chem. Soc. 1998, 120, 1959-1964.
(19) R.C. Mucic, J.J. Storhoff, C.A. Mirkin, R.L. Letsinger, J. Am. Chem. Soc. 1998, 120, 12674-12675.
(20) L.M. Demers, C.A. Mirkin, R.C. Mucic, RA. Reynolds III, R.L. Letsinger, R. Elghanian, G. Viswanadham, Anal. Chem. 2000, 72, 5535-5541.
(21) T. Brown, D.J.S. Brown, in Oligonucleotides and Analogues (Ed.: F. Eckstein), Oxford University Press, New York, 1991**.**
(22) L.A. Chrisey, G.U. Lee, and C.E. O'Ferral, Nucl. Acids. Res. 1996, 24, 3031-3039.
(23) Nicewarner-Pena, S.R. Freeman, R.G.; Reiss, B.D.; He, L.; Pena, D.J.; Walton, I.D.; Cromer, R.; Keating, C.D.; Natan M.J. Science 2001, 294, 137.
(24) Ferguson, J.A.; Steemers, F.J.; Walt, D.R. Anal. Chem. 2000, 72, 5618.
(25) Han, M.; Gao, X.; Nie, S. Nature biotech. 2001, 19, 631.

## Claims

1. A method for detecting for the presence of one or more target analytes in a sample comprising:
providing one or more types of particle complex probes, each type of probe comprising a particle having oligonucleotides bound thereto, a DNA barcode, and a oligonucleotide having bound thereto a specific binding complement to a specific target analyte, wherein the specific target analyte and the specific binding complement are members of a specific binding pair, wherein.
(i) the DNA barcode has a sequence having at least two portions,
(ii) at least some of the oligonucleotides attached to the particle have a sequence that is complementary to a first portion of the DNA barcode,
(iii) the oligonucleotides having bound thereto a specific binding complement to the target analyte have a sequence that is complementary to a second portion of the DNA barcode, and
(iv) the DNA barcode in each type of particle complex probe has a sequence that is different and that serves as an identifier for a particular target analyte;
contacting the sample with a particle complex probe under conditions effective to allow specific binding interactions between the analyte and the particle complex probe and to form an aggregated complex in the presence of analyte;
isolating aggregated complexes; and
analyzing the aggregated complexes to determine the presence of one or more DNA barcodes having different sequences.

2. The method according to claim 1 wherein said isolating and analyzing the aggregated complexes comprises the steps of:
isolating the aggregated complex and subjecting the aggregated complex to conditions effective to dehybridize the aggregated complex and to release the DNA barcode;
isolating the DNA barcode; and
detecting for the presence of DNA barcode.

3. A method according to claim 1 including the step of isolating the DNA barcodes; and
detecting for the presence of one or more DNA barcodes having different sequences, to determine the presence of a specific target analyte in the sample.

4. A method according to Claim 1, wherein the target analyte and the specific binding complement are members of an antigen-antibody pair.

5. A method according to Claim 1, wherein the specific binding complement bound to the oligonucleotide is a hapten.

6. The method of any one of the preceding claims wherein the particles are nanoparticles.

7. The method of any one of the preceding claims wherein the particles are metal, semiconductor, insulator, or magnetic nanoparticles.

8. The method of any one of the preceding claims wherein wherein the particles are gold nanoparticles.

9. The method of any one of the preceding claims wherein the DNA barcodes are detected using a substrate having oligonucleotides attached thereto, the oligonucleotdes having a sequence complementary to the sequence of a portion of the DNA barcode.

10. The method of claim 9 wherein, wherein the substrate has a plurality of types of oligonucleotides attached thereto in an array to allow for the detection of multiple portions of a single type of DNA barcode, the detection of multiple different DNA barcodes, or both.

11. The method of any one of the preceding claims wherein further comprising
providing a substrate having a plurality of types of oligonucleotides attached thereto in an array to allow for the detection of multiple portions of a single type of DNA barcode, the detection of multiple different DNA barcodes, or both;
providing a nanoparticle having oligonucleotides bound thereto, wherein a portion of the oligonucleotides have a sequence that is complementary to a portion of a DNA barcode;
contacting the DNA barcodes with the substrate and the nanoparticles under conditions effective for hybridization of at least a first portion of the DNA barcodes with a complementary oligonucleotide bound to the substrate and the second portion of the DNA barcodes with some of the oligonucleotides bound to the nanoparticles; and
observing a detectable change.

12. The method of claim 11 wherein the detectable change is the formation of dark areas on the substrate.

13. The method according to claim 11, wherein the detectable change is observed with an optical scanner.

14. The method according to claim 11, wherein the substrate is contacted with silver stain to produce the detectable change.

15. The method according to claim 11, wherein the DNA barcodes are contacted with the substrate so that the DNA barcodes hybridize with complementary oligonucleotides bound to the substrate and then the DNA barcodes bound to the substrate is contacted with the nanoparticles having oligonucleotides bound thereto so that at least some of the oligonucleotides bound to the nanoparticles hybridize with a portion of the sequence of the DNA barcodes on the substate.

16. The method according to claim 10, wherein the DNA barcodes are contacted with the nanoparticles having oligonucleotides bound thereto so that at least some of the oligonucleotides bound to the nanoparticles hybridize with a portion of the sequence of the DNA barcodes; and contacting the DNA barcodes bound to the nanoparticles with the substrate so that a portion of the sequence of the DNA barcodes bound to the nanoparticles hybridizes with complementary oligonucleotides bound to the substrate.

17. A system for detecting one or more target analytes in a sample comprising:
one or more types of particle complex probes, each particle complex probe comprising a particle having oligonucleotides bound thereto, a DNA barcode, and an oligonucleotide having bound thereto a specific binding complement to a specific target analyte, wherein the specific target analyte and the specific binding complement are members of a specific binding pair, and wherein
(i) the DNA barcode has a sequence having at least two portions,
(ii) at least some of the oligonucleotides attached to the particle have a sequence that is complementary to a first portion of a DNA barcode,
(iii) the oligonucleotide having bound thereto a specific binding complement have a sequence that is complementary to a second portion of a DNA barcode, and
(iv) the DNA barcode in each type of particle complex probe has a sequence that is different and that serves as an identifier for a particular target analyte.

18. The system according to claim 17, wherein the particle comprises a nanoparticle.

19. The system according to claim 18, wherein the particles are metal, semiconductor, insulator, or magnetic nanoparticles.

20. The system according to claim 18, wherein the particles are gold nanoparticles.

21. A kit for detecting a target analyte in a sample, comprising at least one container including a particle complex probe system according to Claim 17 and optionally includes a substrate for observing a detectable change.

22. A kit according to claim 21, which includes a plurality of DNA barcodes, wherein the DNA barcode in each type of particle complex probe has a sequence that is different and that serves as an identifier for a particular target analyte.

23. A kit according to claim 21, which includes a first container including a particle probe comprising a particle having oligonucleotides bound thereto and a DNA barcode having a sequence of at least two portions, wherein at least some of the oligonucleotides attached to the particle have a sequence that is complementary to a first portion of a DNA barcode;
and a second container including an oligonucleotide having a sequence that is complementary to a second portion of the DNA barcode, the oligonucleotide having a moiety that can be used to covalently link a specific binding pair complement of a target analyte.

24. A kit according to claim 21 for the detection of multiple target analytes in a sample, wherein the kit includes at least two pairs of containers,
wherein the first container of each pair includes particle complex probes having particles having oligonucleotides bound thereto and a DNA barcode having a sequence of at least two portions, wherein at least some of the oligonucleotides bound to the particles have a sequence that is complementary to a first portion of a DNA barcode having at least two portions; and
the second container of each pair contains an oligonucleotide having a sequence that is complementary to a second portion of the DNA barcode, the oligonucleotide having a moiety that can be used to covalently link a specific binding pair complement of a target analyte,
wherein the DNA barcode for type of particle complex probe has a sequence that is different and that serves as an identifer for a target analyte and wherein the kit optionally include a substrate for observing a detectable change.

25. A kit according to any one of claims 21 to 24, wherein the particle comprises a nanoparticle.

26. The kit according to claim 25, wherein the particles are metal, semiconductor, insulator, or magnetic nanoparticles.

27. The kit according to claim 26, wherein the particles are gold nanoparticles.

## Patentansprüche

1. Verfahren zur Detektion der Gegenwart eines oder mehrere Zielanalyte in einer Probe umfassend:
Bereitstellen einer oder mehrerer Arten von komplexen Partikelsonden, wobei jede Art von Sonde ein Partikel, an welches Oligonukleotide gebunden sind, einen DNA Barcode und ein Oligonukleotid umfasst, woran ein spezifisch bindendes Komplement zu einem Zielanalyt gebunden ist, wobei das spezifische Zielanalyt und das spezifisch bindende Komplement Teile eines spezifischen Bindungspaars sind, wobei
(i) der DNA Barcode eine Sequenz mit zumindest zwei Teilen aufweist,
(ii) zumindest einige der an das Partikel gebundenen Oligonukleotide eine Sequenz aufweisen, die komplementär zu einem ersten Teil des DNA Barcodes ist,
(iii) an die Oligonukleotide gebunden ein spezifisch bindendes Komplement zu dem Zielanalyt vorliegt, welches eine Sequenz hat, die komplementär zu einem zweiten Teil des DNA Barcodes ist, und
(iv) der DNA Barcode bei jeder Art der komplexen Partikelsonde eine unterschiedliche Sequenz aufweist und die als Kennung für ein bestimmtes Zielanalyt dient;
In Kontakt bringen der Probe mit einer komplexen Partikelsonde unter Bedingungen, die effektiv sind, spezifische Bindungswechselwirkungen zwischen dem Analyt und der komplexen Partikelprobe zu erlauben und es erlauben, bei Anwesenheit des Analyts einen aggregierten Komplex zu bilden;
Isolieren der aggregierten Komplexe; und
Analysieren der aggregierten Komplexe, um die Gegenwart von einem oder mehreren DNA Barcodes mit unterschiedlichen Sequenzen zu bestimmen.

2. Das Verfahren nach Anspruch 1, wobei das Isolieren und Analysieren der aggregierten Komplexe die Schritte umfasst:
Isolieren der aggregierten Komplexe und Aussetzen der aggregierten Komplexe an Bedingungen, die wirksam sind, die Hybridisierung der aggregierten Komplexe zu dehybridisieren und den DNA Barcode freizusetzen;
Isolieren des DNA Barcodes; und
Detektieren der Gegenwart von DNA Barcode.

3. Das Verfahren nach Anspruch 1, einschließlich des Schritts des Isolierens der DNA Barcodes; und
Detektieren der Gegenwart von einem oder mehreren DNA Barcodes mit unterschiedlichen Sequenzen, um die Gegenwart eines spezifischen Zielanalyten in der Probe zu bestimmen.

4. Das Verfahren nach Anspruch 1, wobei das Zielanalyt und das spezifische Bindungskomplement Teile eines Antigen-Antikörper Paars sind.

5. Das Verfahren nach Anspruch 1, wobei das spezifische Bindungskomplement, welches an das Oligonukleotid gebunden ist, ein Hapten ist.

6. Das Verfahren nach einem der vorstehenden Ansprüche, wobei die Partikel Nanopartikel sind.

7. Das Verfahren nach einem der vorstehenden Ansprüche, wobei die Partikel Metall-, Halbleiter-, Isolator-, oder magnetische Nanopartikel sind.

8. Das Verfahren nach einem der vorstehenden Ansprüche, wobei die Partikel Goldnanopartikel sind.

9. Das Verfahren nach einem der vorstehenden Ansprüche, wobei die DNA Barcodes unter Verwendung eines Substrats mit daran gebundenen Oligonukleotiden detektiert werden, wobei die Oligonukleotide eine Sequenz aufweisen, die komplementär zu der Sequenz eines Teils des DNA Barcodes ist.

10. Das Verfahren nach Anspruch 9, wobei das Substrat eine Vielzahl von Arten von daran in einem Array gebundenen Oligonukleotiden aufweist, um die Detektion von mehreren Teilen einer einzelnen Art eines DNA Barcodes, die Detektion mehrerer unterschiedlicher DNA Barcodes oder beidem zu erlauben.

11. Das Verfahren nach einem der vorstehenden Ansprüche, weiterhin umfassend Bereitstellen eines Substrats, welches eine Vielzahl von daran in einem Array gebundene Oligonukleotide aufweist, um die Detektion von mehreren Teilen einer einzelnen Art eines DNA Barcodes, die Detektion mehrerer unterschiedlicher DNA Barcodes oder beidem zu ermöglichen;
Bereitstellen eines Nanopartikels, welches daran gebundene Oligonukleotide aufweist, wobei ein Teil der Oligonukleotide eine Sequenz aufweist, die komplementär zu einem Teil des DNA Barcodes ist;
In Kontakt bringen der DNA Barcodes mit dem Substrat und den Nanopartikeln unter Bedingungen, die wirksam sind, eine Hybridisierung von zumindest einem ersten Teil der DNA Barcodes mit einem komplementären an das Substrat gebundenen Oligonukleotid und eine Hybridisierung eines zweiten Teils der DNA Barcodes mit einigen der an die Nanopartikel gebundenen Oligonukleotide herbeizuführen; und
Beobachten einer detektierbaren Änderung.

12. Das Verfahren nach Anspruch 11, wobei die detektierbare Änderung die Bildung von dunklen Bereichen auf dem Substrat ist.

13. Das Verfahren nach Anspruch 11, wobei die detektierbare Änderung mit einem optischen Scanner beobachtet wird.

14. Das Verfahren nach Anspruch 11, wobei das Substrat mit Silberfarbung in Kontakt gebracht wird, um eine detektierbare Änderung hervorzurufen.

15. Das Verfahren nach Anspruch 11, wobei die DNA Barcodes so mit dem Substrat in Kontakt gebracht werden, dass die DNA Barcodes mit komplementären Oligonukleotiden hybridisieren, die an das Substrat gebunden sind und danach die an das Substrat gebundenen DNA Barcodes mit Nanopartikeln in Kontakt gebracht werden, die daran gebundene Oligonukleotide aufweisen, so dass zumindest einige der an die Nanopartikel gebundenen Oligonukleotide mit einem Teil der Sequenz der DNA Barcodes auf dem Substrat hybridisieren.

16. Das Verfahren nach Anspruch 10, wobei die DNA Barcodes mit den Nanopartikeln in Kontakt gebracht werden, die daran gebundene Oligonukleotide aufweisen, so dass zumindest einige der an die Nanopartikel gebundenen Oligonukleotide mit einem Teil der Sequenz der DNA Barcodes hybridisieren; und in Kontakt bringen der an die Nanopartikel gebundenen DNA Barcodes mit dem Substrat, so dass ein Teil der Sequenz der an die Nanopartikel gebundenen DNA Barcodes mit den komplementären Oligonukleotiden hybridisiert, die an das Substrat gebunden sind.

17. System zur Detektion eines oder mehrere Zielanalyten in einer Probe, umfassend:
eine oder mehrere Arten von komplexen Partikelsonden, wobei jede Art von Sonde ein Partikel, an welches Oligonukleotide gebunden sind, einen DNA Barcode und ein Oligonukleotid umfasst, woran ein spezifisch bindendes Komplement zu einem Zielanalyt gebunden ist, wobei das spezifische Zielanalyt und das spezifisch bindende Komplement Teile eines spezifischen Bindungspaars sind, wobei
(i) der DNA Barcode eine Sequenz mit zumindest zwei Teilen aufweist,
(ii) zumindest einige der an das Partikel gebundenen Oligonukleotide eine Sequenz aufweisen, die komplementär zu einem ersten Teil des DNA Barcodes ist,
(iii) an das Oligonukleotid gebunden ein spezifisch bindendes Komplement zu dem Zielanalyt vorliegt, welches eine Sequenz hat, die komplementär zu einem zweiten Teil des DNA Barcodes ist, und
(iv) der DNA Barcode bei jeder Art der komplexen Partikelsonde eine unterschiedliche Sequenz aufweist und die als Kennung für ein bestimmtes Zielanalyt dient;

18. Das System nach Anspruch 17, wobei das Partikel ein Nanopartikel umfasst.

19. Das System nach Anspruch 18, wobei die Partikel Metall-, Halbleiter-, Isolator-, oder magnetische Nanopartikel sind.

20. Das System nach Anspruch 18, wobei die Partikel Goldnanopartikel sind.

21. Kit zur Detektion eines Zielanalyts in einer Probe umfassend zumindest einen Behälter einschließlich eines komplexen Partikelsondensystems nach Anspruch 17 und optional einschließlich eines Substrats zur Beobachtung einer detektierbaren Änderung.

22. Das Kit nach Anspruch 21, welches eine Vielzahl von DNA Barcodes enthält, wobei der DNA Barcode bei jeder Art von komplexer Partikelsonde eine Sequenz aufweist, die unterschiedlich ist und die als eine Kennung für ein bestimmtest Zielanalyt dient.

23. Das Kit nach Anspruch 21, welches einen ersten Behälter einschließt, der eine Partikelsonde enthält, die ein Partikel umfasst, welches ein daran gebundenes Oligonukleotid aufweist und einen DNA Barcode enthält, der eine Sequenz mit zumindest zwei Teilen aufweist, wobei zumindest einige der an das Partikel gebundenen Oligonukleotide eine Sequenz aufweisen, die komplementär zu einem ersten Teil eines DNA Barcodes ist;
und einen zweiten Behälter einschließt, der ein Oligonukleotid enthält, das eine Sequenz aufweist, die komplementär zu einem zweiten Teil des DNA Barcodes ist, wobei das Oligonukleotid eine Gruppe aufweist, die verwendet werden kann, um ein spezifisches Bindungspaarkomplement eines Zielanalyten kovalent zu verbinden.

24. Das Kit nach Anspruch 21 zur Detektion von mehreren Zielanalyten in einer Probe, wobei das Kit zumindest zwei Paare von Behältern einschließt,
wobei der erste Behälter eines jeden Paars komplexe Partikelsonden mit Partikeln enthält, die daran gebundene Oligonukleotide aufweisen und einen DNA Barcode enthält, der eine Sequenz aus zumindest zwei Teilen aufweist, wobei zumindest einige der Oligonukleotide, welche an die Partikel gebunden vorliegen, eine Sequenz aufweisen, die komplementär zu einem ersten Teil eines DNA Barcodes ist, der zumindest zwei Teile aufweist; und
wobei der zweite Behälter eines jeden Paars ein Oligonukleotid enthält, welches eine Sequenz aufweist, die komplementär zu einem zweiten Teil des DNA Barcodes ist, wobei das Oligonukleotid eine Gruppe aufweist, die verwendet werden kann, um ein spezifisches Bindungspaarkomplement eines Zielanalyten kovalent zu verbinden,
wobei der DNA Barcode einer jeden Art von komplexer Partikelsonde eine Sequenz aufweist, die unterschiedlich ist und die als Kennung für ein bestimmtes Zielanalyt dient, und wobei das Kit optional ein Substrat für die Beobachtung der detektierbaren Änderung einschließt.

25. Das Kit nach einem der Ansprüche 21 bis 24, wobei die Partikel Nanopartikel umfassen.

26. Das Kit nach Anspruche 25, wobei die Partikel Metall-, Halbleiter-, Isolator-, oder magnetische Nanopartikel sind.

27. Das Kit nach Anspruche 26, wobei die Partikel Goldnanopartikel sind.

## Revendications

1. Méthode de détection de la présence d'un ou plusieurs analytes cibles dans un échantillon comprenant:
la fourniture d'un ou plusieurs types de sondes complexes à particules, chaque type de sonde comprenant une particule ayant des oligonucléotides liés à celle-ci, un ADN code barre, et un oligonucléotide ayant lié à celui-ci un complément à liaison spécifique d'un analyte cible spécifique, dans laquelle l'analyte cible spécifique et le complément à liaison spécifique sont membres d'une paire de liaison spécifique, dans laquelle
(i) l'ADN code barre a une séquence ayant au moins deux portions,
(ii) au moins certains des oligonucléotides attachés à la particule ont une séquence qui est complémentaire d'une première portion de l'ADN code barre,
(iii) les oligonucléotides ayant lié à ceux-ci un complément à liaison spécifique de l'analyte cible ont une séquence qui est complémentaire d'une seconde portion de l'ADN code barre, et
(iv) l'ADN code barre dans chaque type de sonde complexe à particules a une séquence qui est différente et qui sert d'identificateur pour un analyte cible particulier ;
la mise en contact de l'échantillon avec une sonde complexe à particules dans des conditions efficaces pour permettre des interactions de liaison spécifiques entre l'analyte et la sonde complexe à particules et pour former un complexe agrégé en présence d'un analyte ;
l'isolement de complexes agrégés ; et
l'analyse des complexes agrégés pour déterminer la présence d'un ou plusieurs ADN code barre ayant différentes séquences.

2. Méthode selon la revendication 1 dans laquelle lesdits isolement et analyse des complexes agrégés comprennent les étapes de :
isolement du complexe agrégé et soumission des complexes agrégés à des conditions efficaces pour déshybrider le complexe agrégé et pour libérer l'ADN code barre ;
isolement de l'ADN code barre ; et
détection de la présence de l'ADN code barre.

3. Méthode selon la revendication 1 incluant l'étape d'isolement des ADN code barre ; et
la détection de la présence d'un ou plusieurs ADN code barre ayant différentes séquences, pour déterminer la présence d'un analyte cible spécifique dans l'échantillon.

4. Méthode selon la revendication 1, dans laquelle l'analyte cible et le complément à liaison spécifique sont membres d'une paire antigène-anticorps.

5. Méthode selon la revendication 1, dans laquelle le complément à liaison spécifique lié à l'oligonucléotide est un haptène.

6. Méthode de l'une quelconque des revendications précédentes dans laquelle les particules sont des nanoparticules.

7. Méthode de l'une quelconque des revendications précédentes dans laquelle les particules sont des nanoparticules métalliques, semi-conductrices, isolantes ou magnétiques.

8. Méthode de l'une quelconque des revendications précédentes dans laquelle les particules sont des nanoparticules d'or.

9. Méthode de l'une quelconque des revendications précédentes dans laquelle les ADN code barre sont détectés en utilisant un substrat ayant des oligonucléotides attachés à celui-ci, les oligonucléotides ayant une séquence complémentaire de la séquence d'une portion de l'ADN code barre.

10. Méthode de la revendication 9, dans laquelle le substrat a une pluralité de types d'oligonucléotides attachés à celui-ci dans un réseau pour permettre la détection de portions multiples d'un seul type d'ADN code barre, la détection de multiples ADN code barre différents, ou les deux.

11. Méthode de l'une quelconque des revendications précédentes contenant en outre
la fourniture d'un substrat ayant une pluralité de types d'oligonucléotides attachés à celui-ci dans un réseau pour permettre la détection de portions multiples d'un seul type d'ADN code barre, la détection de multiples ADN code barre différents, ou les deux ;
la fourniture d'une nanoparticule ayant des oligonucléotides attachée à celle-ci, dans laquelle une portion des oligonucléotides ont une séquence qui est complémentaire d'une portion d'un ADN code barre ;
la mise en contact des ADN code barre avec le substrat et les nanoparticules dans des conditions efficaces pour l'hybridation d'au moins une première portion des ADN code barre avec un oligonucléotide complémentaire lié au substrat et de la seconde portion des ADN code barre avec certains des oligonucléotides liés aux nanoparticules ; et
l'observation d'un changement détectable.

12. Méthode de la revendication 11 dans laquelle le changement détectable est la formation de zones sombres sur le substrat.

13. Méthode selon la revendication 11, dans laquelle le changement détectable est observé avec un scanner optique.

14. Méthode selon la revendication 11, dans laquelle le substrat est mis en contact avec un colorant à l'argent pour produire le changement détectable.

15. Méthode selon la revendication 11, dans laquelle les ADN code barre sont mis en contact avec le substrat de façon que les ADN code barre s'hybrident avec des oligonucléotides complémentaires liés au substrat et ensuite l'ADN code barre lié au substrat est mis en contact avec les nanoparticules ayant des oligonucléotides liés à celles-ci de façon qu'au moins certains des oligonucléotides liés aux nanoparticules s'hybrident avec une portion de la séquence des ADN codes barres sur le substrat.

16. Méthode selon la revendication 10, dans laquelle les ADN code barre sont mis en contact avec les nanoparticules ayant des oligonucléotides liés à celles-ci de façon qu'au moins certains des oligonucléotides liés aux nanoparticules s'hybrident à une portion de la séquence des ADN code barre ; et la mise en contact des ADN codes barres liés aux nanoparticules avec le substrat de façon qu'une portion de la séquence des ADN code barre liés aux nanoparticules s'hybride avec des oligonucléotides complémentaires liés au substrat.

17. Système pour détecter un ou plusieurs analytes cibles dans un échantillon comprenant:
un ou plusieurs types de sondes complexes à particules, chaque sonde complexe à particules comprenant une particule ayant des oligonucléotides liés à celle-ci, un ADN code barre et un oligonucléotide ayant lié à celui-ci un complément à liaison spécifique d'un analyte cible spécifique, dans lequel l'analyte cible spécifique et le complément à liaison spécifique sont membres d'une paire de liaison spécifique, et dans lequel
(i) l'ADN code barre a une séquence ayant au moins deux portions,
(ii) au moins certains des oligonucléotides attachés à la particule ont une séquence qui est complémentaire d'une première portion d'un ADN code barre,
(iii) l'oligonucléotide ayant lié à celui-ci un complément à liaison spécifique a une séquence qui est complémentaire d'une seconde portion d'un ADN code barre, et
(iv) l'ADN code barre dans chaque type de sonde complexe à particules a une séquence qui est différente et qui sert d'identificateur pour un analyte cible particulier.

18. Système selon la revendication 17, dans lequel la particule comprend une nanoparticule.

19. Système selon la revendication 18, dans lequel les particules sont des nanoparticules métalliques, semi-conductrices, isolantes, ou magnétiques.

20. Système selon la revendication 18, dans lequel les particules sont des nanoparticules d'or.

21. Kit de détection d'un analyte cible dans un échantillon, incluant au moins un récipient comprenant un système de sondes complexes à particules selon la revendication 17 et qui éventuellement inclut un substrat pour observer un changement détectable.

22. Kit selon la revendication 21, qui inclut une pluralité d'ADN code barre, dans lequel l'ADN code barre dans chaque type de sonde complexe à particules a une séquence qui est différente et qui sert d'identificateur pour un analyte cible particulier.

23. Kit selon la revendication 21, qui inclut un premier récipient incluant une sonde à particules comprenant une particule ayant des oligonucléotides liés à celle-ci et un ADN code barre ayant une séquence d'au moins deux portions, dans laquelle au moins certains des oligonucléotides attachés à la particule ont une séquence qui est complémentaire d'une première portion d'un ADN code barre ;
et un second récipient incluant un oligonucléotide ayant une séquence qui est complémentaire d'une seconde portion de l'ADN code barre, l'oligonucléotide ayant une partie qui peut être utilisée pour lier de manière covalente une paire à liaison spécifique complément d'un analyte cible.

24. Kit selon la revendication 21 pour la détection d'analytes cibles multiples dans un échantillon, dans lequel le kit inclut au moins deux paires de récipients,
dans lequel le premier récipient de chaque paire inclut des sondes complexes à particules ayant des particules ayant des oligonucléotides liés à celles-ci et un ADN code barre ayant une séquence d'au moins deux portions, dans lequel au moins certains des oligonucléotides liés aux particules ont une séquence qui est complémentaire d'une première portion d'un ADN barre code ayant au moins deux portions ; et
le second récipient de chaque paire contient un oligonucléotide ayant une séquence qui est complémentaire d'une seconde portion de l'ADN code barre, l'oligonucléotide ayant une partie qui peut être utilisée pour lier de manière covalente une paire à liaison spécifique complément d'un analyte cible,
dans lequel l'ADN code barre pour un type de sonde complexe à particules a une séquence qui est différente et qui sert d'identificateur pour un analyte cible et dans lequel le kit inclut éventuellement un substrat pour observer un changement détectable.

25. Kit selon l'une quelconque des revendications 21 à 24, dans lequel la particule comprend une nanoparticule.

26. Kit selon la revendication 25, dans lequel les particules sont des nanoparticules métalliques, semi-conductrices, isolantes ou magnétiques.

27. Kit selon la revendication 26, dans lequel les particules sont des nanoparticules d'or.


### SEQUENCE LISTING

<110> Mirkin, Chad A.
   Park, So-Jung
   Nam, Jwa-Min
<120> BIO-BARCODES BASED ON OLIGONUCLEOTIDE-MODIFIED NANOPARTICLES
<130> 01-1705-A
<140> 10/108,211
   <141> 2002-03-27
<150> 60/192,699
   <151> 2001-03-28
<150> 60/350,560
   <151> 2001-11-13
<160> 8
<170> Microsoft Word 2000 <210> 1
   <211> 15
   <212> DNA
   <213> Artificial Sequence
<220>
   <223> Description of Artificial Sequence:random synthetic sequence
<400> 1
   ataactagaa cttga 15
<210> 2
   <211> 12
   <212> DNA
   <213> Artificial Sequence
<220>
   <223> Description of Artificial Sequence:random synthetic sequence
<400> 2
   ttatctatta tt 12
<210> 3
   <211> 25
   <212> DNA
   <213> Artificial Sequence
<220>
   <223> Description of Artificial Sequence:random synthetic sequence
<400> 3
   aaaaaaaaaa ataactagaa cttga 25
<210> 4
   <211> 25
   <212> DNA
   <213> Artificial Sequence
<220>
   <223> Description of Artificial Sequence:random synthetic sequence
<400> 4
   tctgaattga ttacgaaaaa aaaaa 25
<210> 5
   <211> 30
   <212> DNA
   <213> Artificial Sequence
<220>
   <223> Description of Artificial Sequence:random synthetic sequence
<400> 5
   cgtaatcaat tcagatcaag ttctagttat 30
<210> 6
   <211> 22
   <212> DNA
   <213> Artificial Sequence
<220>
   <223> Description of Artificial Sequence:random synthetic sequence
<400> 6
   aaaaaaaaaa ttatctatta tt 22
<210> 7
   <211> 22
   <212> DNA
   <213> Artificial Sequence
<220>
   <223> Description of Artificial Sequence:random synthetic sequence
<400> 7
   ttatatgatt ataaaaaaaa aa 22
<210> 8
   <211> 24
   <212> DNA
   <213> Artificial Sequence
<220>
   <223> Description of Artificial Sequence:random synthetic sequence
<400> 8
   ataatcatat aaaataatag ataa 24